(19) Europäisches Patentamt
European Patent Office
Office européen des brevets

(11) EP 4 714 428 A1

(12) EUROPEAN PATENT APPLICATION

(43) Date of publication:
**25.03.2026 Bulletin 2026/13**

(21) Application number: **24202082.4**

(22) Date of filing: **24.09.2024**

(51) International Patent Classification (IPC):
***A61K 8/73*** *(2006.01)* ***A61K 31/722*** *(2006.01)*
***A61P 1/02*** *(2006.01)* ***A61Q 11/00*** *(2006.01)*

(52) Cooperative Patent Classification (CPC):
**A61Q 11/00; A61K 8/736; A61K 31/722; A61P 1/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **SkyLab AG**
**1066 Epalinges (CH)**

(72) Inventors:
- **Belous, Elena Yur'evna**
  **121309 MOSCOW (RU)**
- **Ivanova, Angelina Dmitrievna**
  **London, NW61NE (GB)**

(74) Representative: **Glawe, Delfs, Moll Partnerschaft mbB**
**Hopfenmarkt 33**
**20457 Hamburg (DE)**

# (54) CHITOSAN HYDROCHLORIDE DENTAL FORMULATION FOR ENHANCED ENAMEL REINFORCEMENT, TARGETED ORAL PATHOGEN BIOFILM DISRUPTION, AND DURABLE ACID-RESISTANT ENAMEL PROTECTION

(57) The invention relates to the use of chitosan hydrochloride as a tooth enamel remineralizing agent and dental or oral care products comprising chitosan hydrochloride for medical and aesthetic use.

Graphic of a visual comparison between MIC and MBIC

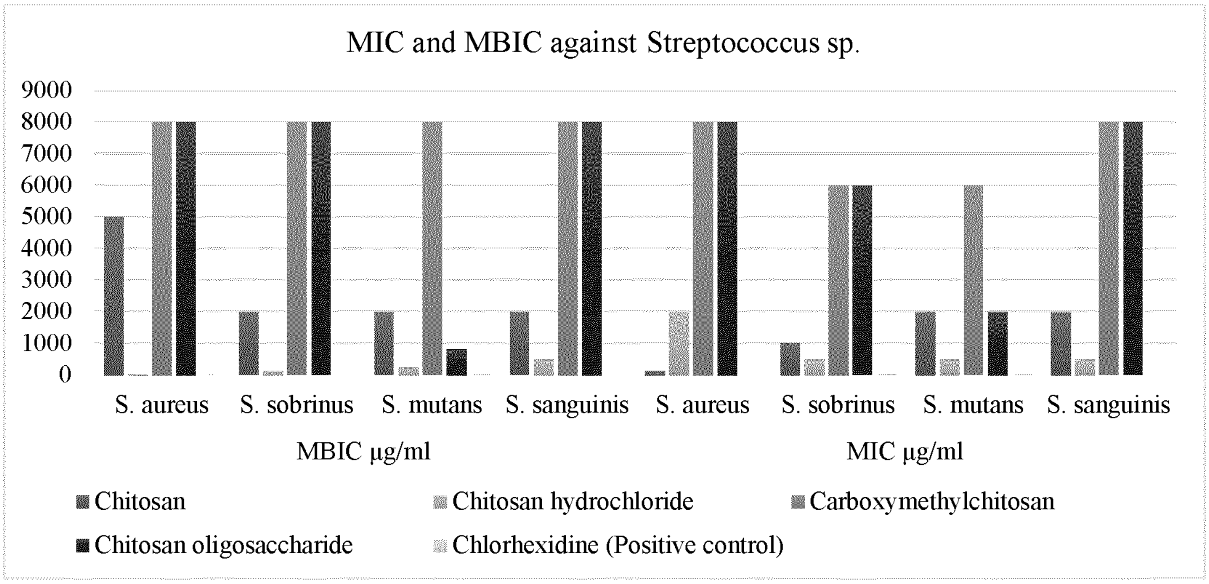


Fig. 1



Processed by Luminess, 75001 PARIS (FR)

## Description

### FIELD OF THE INVENTION

The present invention is related to the novel chitosan hydrochloride uses and can be used in the field of dental care preparations.

### BACKGROUND OF THE INVENTION

Enamel remineralization is a natural process of healing of the lesions at early stage of dental caries/early erosive wear of teeth consisting in deposition of calcium, phosphate and other ions facilitated by the saliva proteins in the voids of demineralized enamel [1-4].

Over the recent years better understanding of physical-chemical and biological mechanisms of mineralization has influenced the development of a number of remineralization methods, which are beyond the fluoride-mediated remineralization [3]. Some fluoride-free remineralization systems are available, which are now in the testing phase or already at the market.

Fluoride exhibits a very beneficial effect on the process of dental enamel mineralization. Conventional fluoride-based treatment plays an essential role in enamel restoration, because it is very effective in breaking the process of enamel demineralization/degradation, and supports the enamel remineralization [5]. These fluoride properties are conventionally used in some non-fluoride approaches through fluoride addition, for instance to reinforce the hardness of remineralized enamel. Controlled use of fluoride both regulates calcium and phosphorus diffusion right in the area of remineralization, and adjusts the reaction velocity.

However, some risk factors are associated with too frequent treatment/intake of fluorides (for instance, fluorinated water, fluorinated tooth paste, or high fluoride concentrations, which are not adapted to the patient). The former can lead to the development of fluorosis of teeth [6,7], while the latter can lead to fluoride syndrome [8]. Therefore, there is a strong need in development of remineralization systems, which will supplement the fluoride in efficacy or even replace it in future.

The remineralization systems, which are currently under development, are based on dentine proteins amelogenin and phosphoprotein or on synthetic peptides, which are very much alike those salivary proteins and show similar physiological properties. Quite recently statherin peptides were added to this group. All these systems specifically adsorb at remineralization sites and accumulate Ca and P ions from saliva; this process leads to local oversaturation with the above ions and finally to hydroxyapatite crystallization [8,9]. Of note, the antibacterial preparations (histatin-3 peptides) can be introduced to virtually any of these systems [10,11]. Some of these remineralization strategies are already at the development stage, especially those belonging to the group of systems based on calcium phosphate. These systems can be used as a treatment of early caries lesions and at the early stage of erosive wear of teeth. However, some of the described systems are not allowed to be used in strict clinical conditions, because they have not been appropriately tested, have side effects or exhibit controversial therapeutic efficacy.

Internal (endogenous) approaches with calcium phosphate (iCP). The most promising iCP-systems include DSS peptides, obtained from dentine phosphoprotein (DPP), polypeptides and amelogenin-based derivatives (for instance, leucin-rich ameogenin peptides, enamel matrix (amelogenin) derivative, so called P11-4 peptide (amelogenin inspired), and poly(amidoamine) dendrimers [8,12]. Unfortunately, statherin-based strategies are rarely reported, perhaps, due to the fact that the other systems currently are more promising. Remineralization with the use of P11-4 peptide is the only iCP strategy, which is currently tested in the clinical trials.

Peptides belonging to the group of dentine phosphoproteins (DPP). In this group the peptides comprising eight repetitive sequences of aspartate-serine-serine (8DSS) are the most active facilitators of biomineralization [13]. 8DSS peptides adsorb on surface of the hydroxyapatite forming strong bonds rather than just securely bind free $Ca^{2+}$ and $PO_4^{3-}$ ions in saliva [13,14]. Therefore, 8DSS peptides satisfy two conditions: a) they can block the demineralization process and dissolution of $Ca^{2+}$ and $PO_4^{3-}$ ions in the environment (due to adsorption on the enamel crystals) and b) they contribute to uptake of said ions from saliva to initiate and regulate the mineralization process. However, the 8DSS peptides were investigated mainly in *in vitro* studies with one above mentioned exception. Therefore, it is not known whether strong binding of said peptides to Ca leads to *in vivo* CaP accumulation and thus to dental calculus deposition. Future investigations will clarify this potential disadvantage. Nevertheless, the 8DSS strategy based on fluoride-free biomineralizing agent is very promising [15].

Brushite (dicalcium phosphate dihydrate, crystalline). This compound is one of the most soluble phases of calcium phosphate and has been commercialized in the dental care products [16,17]. However, brushite was not investigated in *in vivo* studies, and available results of *in vitro* studies do not reflect complex biological processes involved in the lesion remineralization. Furthermore, the clinical trials were not performed. In particular, dental caries progress with the use of brushite was not studied [3].

Calcium-sodium phosphosilicate (CSP, amorphous), bioglass. Bioglass-supported remineralization is achieved due to the ion release, as in the event of crystalline compounds. CSP system is described as both biocompatible and bioactive. In the oral cavity CSP releases calcium and phosphate ions, which support natural remineralization of tooth enamel [18]. Since CSP can support remineralization being in the other form (for instance, more soluble CSP) or in the absence of polymers or other additives (for instance, in the other tooth paste), further *in vitro* research and clinical trials are needed.

Alkaline or alkaline-earth polyphosphates. So called polyphosphates are among the more recent applications. These compounds are added to the dental care products for partial fluoride replacement. Among the polyphosphates (such as sodium trimetaphosphate (STMP), calcium glycerophosphate or hexametaphosphate [19-21]), STMP is the most efficacious anticariogenic agent, and is on the market as a tooth paste Oral-B Pro-Expert [8]. Dual STMP effect has been reported; it prevents demineralization and contributes to remineralization [22,23].

Over the recent years chitosan has been used as a carrier or stabilizer of calcium phosphates due to its biological properties and additional combination with biomimetic mineralization technology. Composite materials obtained with the use of such approach are widely used for restoration of dental solid tissues, especially for biomimetic enamel and dentin remineralization [24].

Among the chitosan applications chitosan remineralizing capacity can be mentioned, which strengthens the dental tissues and, therefore, acts as a desensitizer in tooth pastes. The use of chitosan was associated with better surgical healing of post-extraction wounds in the oral cavity [25]. In addition, some studies showed the reduction of bacterial biofilm when chitosan was used in dental cements. Chitosan exhibits antibacterial, antifungal, hemostatic, and other systemic properties, making it useful for the drug delivery. Chitosan effectively contributes to the wound healing and initial osteogenesis in the housing of erupted tooth after extraction [26]. Tooth paste with chitosan 5% is effective in reducing the number of bacterial colonies of *Streptococcus mutans* in the case of early childhood caries in children [27]. Chitosan properties, such as biocompatibility, anti-inflammatory effect, and others can provide the promising results in periodontitis treatment or wound healing after the tooth extraction. The chitosan properties, such as biocompaibility, biodegradability, nontoxicity, and antibacterial activity provide great opportunities for the use in various applications [28].

Chitosan inhibits the phosphorus release thus preventing the demineralization process of dental enamel. Demineralization depends both on biopolymer concentration and the duration of biopolymer exposure to enamel. Measurements of micro-hardness can be used as a test for the loss of minerals by dental enamel. Chitosan facilitates inhibition of enamel demineralization thus acting as a barrier protecting from acid penetration [29]. Chitosan is highly efficacious against various fungi and bacteria, and this explains the antibacterial effect demonstrated by the most chitosan-based products and chitosan derivatives, such as micro/nanoparticles, gels and films [30].

The chitosan derivatives are widely used for various biomedical purposes due to unique properties, such as biocompatibility, non-toxicity and gel formation capacity. Carboxymethyl chitosan proved to be biocompatible and showed prophylactic activity preventing formation of Gram-positive and Gram-negative bacterial biofilms. Furthermore, due to abundant carboxy groups carboxymethyl chitosan can bind calcium ions and inhibit the amorphous calcium phosphate transformation into hydroxyapatite crystals, which is a key element for induction of dentine biomimetic remineralization [31].

Chitosan oligosaccharide is an oligomer chitosan ($\beta$-(1$\rightarrow$4)-bound d-glucosamine), produced from chitin degradation. Biological activity of chitosan oligosaccharide including anti-inflammatory activity stimulating the tissue regeneration, and antimicrobial properties have been described in numerous recent studies. This chitosan derivative is also suitable for tissue engineering and drug/gene delivery. Due to these beneficial properties chitosan oligosaccharide and the derivatives thereof have been investigated for use in biomedical, pharmaceutical, nutraceutical, and cosmeceutical applications, which showed promising results [32].

The patent [Lina N., e.a. Material for demineralizing dentin collagen fibril, preparation method and application thereof. Patent US, no. 2020330360A1, 2019] describes the use of water-soluble chitosan and aminocarboxylic acid metal chelating agent for dentin demineralization before dental bonding and restoration. These findings bring into question the use of chitosan for dental remineralization under condition of demonstrated retroactive effect.

Chitosan has low solubility at physiological pH (> 6,0), which puts down to the use thereof in the systems requiring higher solubility [33]. In this view the authors considered water-soluble chitosan forms. In the course experiments the authors found that chitosan hydrochloride was the most efficacious chitosan form in terms of pathogen suppression in the oral cavity. Various laboratories established the following biocompatibility rating: chitosan hydrochloride < chitosan glutamate < chitosan glycol < chitosan lactate < methylpyrrolidone chitosan [34]. The study of chitosan properties including in vitro assays showed that chitosan hydrochloride exhibited antifungal effect [36], and antimicrobial effect [37]. However, in literature no information on chitosan hydrochloride as remineralizing agent and agent producing acid-resistant film can be found. For this reason, the authors decided to study chitosan in this respect based on chitosan nature and properties.

The patent [Noboru K., e.a. Oral compositions. Patent GB, no. 2132889A] describes the use of chitosan and the salts thereof as an efficacious agent against periodontitis, however, the advantages of particular chitosan hydrochloride have not been disclosed. Furthermore, chitosan and chitosan hydrochloride show different levels of remineralizing capacity, and the latter is superior in terms of efficacy. These results have not been disclosed in the prior art.

## SUMMARY OF THE INVENTION

## DETAILED DESCRIPTION OF THE EMBODIMENTS

Investigations

**Example 1.** Investigation of the antimicrobial and biofilm inhibitory activity of chitosans against clinical isolates of bacteria of the genus Streptococcus sp.

Materials and methods

Investigated ingredients

Table 1. Investigated ingredients in the antimicrobial study

| № | Ingredient | CAS |
|---|---|---|
| 1 | Chitosan Oligosaccharide | 148-411-57-8 |
| 2 | Chitosan hydrochloride | 70694-72-3 |
| 3 | Carboxymethyl Chitosan | 83512-85-0 |
| 4 | Chitosan | 9012-76-4 |
| 5 | Chlorhexidine (Positive control) | 55-56-1 |

1.1 Investigation

Investigated ingredients listed in Table 1. Chitosans were dissolved to the required concentrations in phosphate-buffered saline pH = 6.5. Chlorhexidine was used as reference antimicrobial compound.

Culture media. Nutrient medium - LB broth [38] (%): tryptone - 1.0; yeast extract - 0.5; NaCl - 0.5; pH 8.0), which is prepared from dry ready-made media (20 g/l). The medium is sterilized by autoclaving at 121°C for 20 minutes. The agar medium contains an additional 1.5% agar. Next, bovine serum is added up to 5% v/v and glucose up to 1% w/v.

Species. The antibacterial activity of the investigated compounds was evaluated against four strains of bacteria which were purchased from Iranian Research Organization for Science and Technology (IROST) including: Streptococcus mutans (ATCC 35668), Streptococcus sobrinus (ATCC 27607), Streptococcus sanguis (PTCC1449) and Streptococcus aureus (ATCC14576).

All microorganisms were grown in Tryptic Soy Broth (TSB), Blood Agar and Mitis Salivarius Agar (MSA) at 37 °C in a atmosphere containing 5% CO2. Then, growths were confirmed using Gram staining, catalase test, optochin and bacitracin tests. The bacteria were stored at -80 °C until used in the study.

1.1.1 Determination of the MIC of chitosan by serial dilution method against Streptococcus sp.

Minimal inhibitory concentration (MIC) - this is an indicator of the effect of a substance on a bacterial culture, equal to its minimum concentration at which complete inhibition of bacterial growth occurs within 24 hours of incubation. The MIC of antimicrobial drugs was determined by microdilution in a liquid nutrient medium, which consists of assessing cell viability using the resazurin test.

The MIC value was determined by serial dilution method in LB broth medium containing bovine serum (5% v/v) and glucose (1% w/v) using 96-well sterile plates. Dilutions of the test substances were prepared in a nutrient medium.

The test substance was added to the first well in a concentration 2 times higher than the maximum, in a volume of 200 μl, and its concentration was brought to the minimum by serial two-fold dilution. Then the prepared inoculum was added to each well - 100 μl of an overnight culture with a bacterial suspension density with a concentration of $10^6$ CFU/ml, thereby doubling the concentration of the studied compounds. For this purpose, a suspension of $1.5 \times 10^8$ CFU/ml (with visual inspection corresponds to the McFarland turbidity standard of 0.5) was diluted 100 times. Wells that did not contain the test substances were included as a control (culture growth control). In addition, the purity of the nutrient media was monitored. The plates were incubated in a thermostat at 37 °C for 24 hours. Viability was assessed using the resazurin test.

This test is based on a colorimetric assay to assess the metabolic activity of cells. NADPH-dependent cellular oxidoreductase enzymes may reflect the number of viable cells when reducing resazurin to a product that has a purple color.

To do this, 5 μl of resazurin solution (0.1% solution in water) is added to wells with 150 μl of suspension and left for staining for 5 minutes. The formation of a pink color of resazurin indicated that this concentration of the test drug was insufficient to suppress the viability of bacteria. The first lowest concentration of the test substance (from a series of serial dilutions), where the formation of the pink transformation product of resazurin does not occur, was considered the MIC. Since MIC was determined by serial dilutions in broth with a step of 2, differences between adjacent dilutions were not considered significant. Each experiment included positive (broth with growing culture) and negative (broth without growing culture) controls. The MIC of a compound is taken to be its median value obtained in three independent experiments (biological replicates).

1.1.2. Determination of MBIC (minimum biofilm inhibitory concentration) of chitosans

The MBPC value is determined by serial dilution in LB medium with 5% bovine serum and 1% glucose using 96-well sterile plates. Dilutions of the test substances were prepared in a nutrient medium.

The final concentrations of chitosans were 8000, 4000, 2000,1000,500,250,125,62,31 μg/ml, chlorhexidine 250-1 μg/ml.

The test substance was added to the first well in a concentration 2 times higher than the maximum, in a volume of 200 μl, and its concentration was brought to the minimum by serial two-fold dilution. Then the prepared inoculum was added to each well - 100 μl of an overnight culture with a bacterial suspension density with a concentration of $10^6$ CFU/ml, thereby doubling the concentration of the studied compounds. For this purpose, a suspension of $1.5 \times 10^8$ CFU/ml (with visual inspection corresponds to the McFarland turbidity standard of 0.5) was diluted 100 times. Wells that did not contain the test substances were included as a control (culture growth control). In addition, the purity of the nutrient media was monitored. The plates were incubated in a thermostat at 37 °C for 24 hours.

To quantitatively analyze biofilm formation by bacteria, it is stained with crystal violet [39]. To stain biofilms with crystal violet, the liquid culture is removed after 24 hours of incubation, the wells with biofilms are washed twice with phosphate-buffered saline PBS (pH 7.4), and then dried overnight. Then 100 μl of a 0.5% crystal violet solution (Sigma) dissolved in 96% ethanol is added to the wells, followed by a 20-minute incubation. Then, after removing the crystal violet solution from the wells, the plate is washed with PBS 3-4 times. After drying the plates, 1 ml of 96% ethanol is added to the wells to elute crystal violet bound to the biofilm, and the optical density is measured at a wavelength of 600 nm using an Infinite 200 Pro microplate spectrophotometer (Tecan). Wells incubated with a sterile nutrient medium and subjected to all staining manipulations serve as a control. The MBIC of a compound is taken to be its median value obtained in three independent experiments (biological replicates).

1.2 Results

Table 2. Results of the Minimum Inhibitory Concentration for Chitosans against Streptococcus sp.

| | MIC μg/ml | | | |
|---|---|---|---|---|
| | *S. aureus* | *S. sobrinus* | *S. mutans* | *S. sanguinis* |
| Chitosan | 125 | 1000 | 2000 | 2000 |
| Chitosan hydrochloride | 2000 | 500 | 500 | 500 |
| Carboxymethylchitosan | 8 000 | 6000 | 6000 | 8000 |
| Chitosan oligosaccharide | 8 000 | 6000 | 2000 | 8000 |
| Chlorhexidine (Positive control) | 0.5 | 4 | 4 | 8 |

Table 3. Minimum Biofilm Inhibitory Concentration (MBIC) for Chitosans against Streptococcus sp.

| | MBIC μg/ml | | | |
|---|---|---|---|---|
| | *S. aureus* | *S. sobrinus* | *S. mutans* | *S. sanguinis* |
| Chitosan | 5000 | 2000 | 2000 | 2000 |
| Chitosan hydrochloride | 31 | 125 | 250 | 500 |

(continued)

| | MBIC µg/ml | | | |
|---|---|---|---|---|
| | *S. aureus* | *S. sobrinus* | *S. mutans* | *S. sanguinis* |
| Carboxymethylchitosan | 8000 | 8000 | 8000 | 8000 |
| Chitosan oligosaccharide | 8000 | 8000 | 800 | 8000 |
| Chlorhexidine (Positive control) | 1 | 4 | 4 | 4 |

The above data of Tables 2 and 3 are presented graphically in Figure 1.

Conclusion: Among the Chitosan-based compounds tested for antimicrobial and biofilm inhibition properties against various Streptococcus species, Chitosan hydrochloride stands out with the lowest MIC and MBIC values, indicating a superior efficacy compared to other Chitosan derivatives. Chitosan hydrochloride demonstrates promising potential, especially in inhibiting the growth of S. mutans and S. sanguinis, which are critical in dental biofilm formation and subsequent cavity development. These findings suggest that Chitosan hydrochloride could be a focus for future research, with a view to optimize its properties for clinical applications.

Example 2. Determination of antioxidant activity towards ascorbic acid for a liquid sample using the Ferric reducing antioxidant power (FRAP)

2.1 Materials and methods

2.1.1 Investigated ingredients

Table 4. Investigated ingredients in the FRAP assay

| № | Ingredient | CAS |
|---|---|---|
| 1 | Chitosan Oligosaccharide | 148-411-57-8 |
| 2 | Chitosan hydrochloride | 70694-72-3 |
| 3 | Carboxymethyl Chitosan | 83512-85-0 |
| 4 | Chitosan | 9012-76-4 |

For the experiment, 0.2% (w/v) aqueous solutions of water-soluble Chitosan Oligosaccharide, Chitosan Hydrochloride, and Carboxymethyl Chitosan were prepared. Each compound, weighing 0.2 g, was dissolved in 100 mL of distilled or deionized water, with the aid of a magnetic stirrer to ensure complete dissolution. For the preparation of the Chitosan solution, 0.2 g of Chitosan powder was dissolved in 100 mL of 1% acetic acid solution to create a 0.2% (w/v) mixture. Given Chitosan's limited solubility in neutral and alkaline solutions, the acidic environment facilitated dissolution.

2.2 Investigation

The FRAP assay was conducted in alignment with a modified version of the protocol originally delineated by Benzie and Strain (1996) [40]. All utilized reagents were of analytical grade and procured from recognized suppliers to ensure the assay's consistency and reliability. A 300 mM acetate buffer at pH 3.6 was prepared by dissolving 3.1 g of sodium acetate trihydrate (Sigma-Aldrich, St. Louis, MO, USA) in 16 mL of glacial acetic acid (Fisher Scientific, Hampton, NH, USA), with the volume adjusted to 1 L using distilled water. The TPTZ (2,4,6-tripyridyl-s-triazine) solution was made at 10 mM concentration in 40 mM HCl, and a 20 mM solution of $FeCl_3 \cdot 6H_2O$ was also prepared, both sourced from Sigma-Aldrich (St. Louis, MO, USA). For the preparation of the FRAP reagent, acetate buffer, TPTZ solution, and $FeCl_3 \cdot 6H_2O$ solution were combined in a 10:1:1 (v/v/v) ratio. This mixture was subsequently warmed to 37°C in a water bath (Julabo SW23, Julabo GmbH, Seelbach, Germany) immediately prior to the assay to ensure the reaction proceeded under optimal conditions. In the assay, 150 µL of the FRAP reagent was dispensed into each well of a 96-well plate, to which 50 µL of either the prepared sample or ascorbic acid standard was added. The components were thoroughly mixed using a vortex mixer (Vortex-Genie 2, Scientific Industries, Bohemia, NY, USA) to ensure uniformity.

The reaction mixtures were then incubated at 37°C for 30 minutes in a dark environment, allowing the reduction reactions to complete. Post incubation, the absorbance of each mixture was determined at 593 nm using a spectrophotometer (Shimadzu UV-1800, Shimadzu Corporation, Kyoto, Japan).

Absorbance values obtained from blank samples were subtracted from all other readings to correct for any baseline absorbance. The antioxidant capacities of the samples were quantified by comparing their absorbance values to those of an ascorbic acid standard curve, which was plotted under identical conditions.

This procedure was meticulously outlined to detail the execution of the FRAP assay, ensuring the reproducibility and reliability of the measurements of antioxidant capacities in the analyzed samples.

The general formula used to calculate the antioxidant capacity of the samples in the assay is as follows:

$$Antioxidant\ Capacity = \frac{(Absorbance\ of\ Standart - Absorbance\ of\ Blank)}{(Assorbance\ of\ Sample - Absorbance\ of\ Blank)} \times Concentration\ of\ Standart$$

Absorbance of Sample is the measured absorbance of the reaction mixture containing the test sample.

Absorbance of Blank is the absorbance of the reaction mixture containing all reagents except the sample or standard (negative control).

Absorbance of Standard is the measured absorbance of the reaction mixture containing a known concentration of the standard antioxidant (ascorbic acid).

Concentration of Standard is the known concentration of ascorbic acid used in the assay.

2.3 Result

Table 5. Results of the Antioxidant Capacity for investigated ingredients

| Ingredient | Antioxidant Capacity (Relative to Vitamin C), % |
|---|---|
| Chitosan Oligosaccharide (0.2%) | 106.3 ($p < 0.05$) |
| Chitosan hydrochloride (0.2%) | 114.6 ($p < 0.05$) |
| Carboxymethyl Chitosan (0.2%) | 106.6 ($p < 0.05$) |
| Chitosan (0.2%) | 81 ($p < 0.05$) |
| Ascorbic Acid | 100 |

In this study, Chitosan derivatives were evaluated for their antioxidant capacities against Ascorbic acid (100%). Notably, Chitosan Hydrochloride emerged as the standout, exhibiting the highest antioxidant capacity at 114.6% relative to ascorbic acid. This highlights its potential as a superior antioxidant, surpassing not only ascorbic acid but also other Chitosan derivatives like Chitosan Oligosaccharide (106.3%) and Carboxymethyl Chitosan (106.6%). Chitosan itself showed respectable activity at 81%, underscoring the overall efficacy of Chitosan-based compounds in antioxidant applications.

**Experiment 3.** In Vitro Study to Compare the Ability of Chitosan Versus Chitosan Hydrochloride to Repair and Protect Demineralised Enamel Samples.

3.1 Study design

Enamel blocks were sectioned from bovine teeth, lapped plane-parallel, and highly polished. The baseline surface microhardness of the enamel samples was measured. The samples were then stratified between the treatment groups based on the baseline surface microhardness values.

The enamel samples were artificially demineralised by immersing them in a demineralisation solution for 60 minutes. Following demineralisation, the surface microhardness of the enamel samples was remeasured.

The enamel samples in each treatment group were treated for 16 hours. Following treatment, the surface microhardness of each treated enamel sample was remeasured.

The percentage of surface microhardness recovery (%SMHR) achieved by the test products was calculated and used to compare the ability of the test products to repair the demineralised enamel samples.

Following this, 3 treatment groups and the positive and negative control samples were placed into artificial saliva for 1 minute and then subjected to a 1-hour exposure to a solution containing 1% citric acid (pH 3).

Following the acid challenge, the surface microhardness of each enamel sample was remeasured. The percentage of relative erosion resistance (%RER) achieved by the test products was calculated and used to compare the ability of the test products to repair the demineralised enamel samples.

3.2 Material and methods

3.2.1 Materials

Table 6. Investigated ingredients in %SMHR and %RER studies

| № | Ingredient | CAS |
|---|---|---|
| 1 | Chitosan hydrochloride | 70694-72-3 |
| 2 | Chitosan | 9012-76-4 |
| 3 | Sodium fluoride (Positive control) | 7681-49-4 |
| 4 | Deionised water (Negative control) | 7732-18-5 |

Table 7. Treatments groups in %SMHR and %RER studies

| Treatment Group | Treatment |
|---|---|
| 1 | Chitosan hydrochloride (0.25 %) |
| 2 | Chitosan hydrochloride (0.5%) |
| 3 | 1450 ppm Fluoride (Positive control) |
| 4 | Deionised water (Negative control) |
| 5 | Chitosan 0.5% |

All test solutions (Groups 1 to 5) were prepared in accordance with the analytical protocol, with the test solutions prepared using artificial saliva.

The artificial saliva recipe used to prepare the test solutions is shown below: 400 mg Sodium Chloride (NaCl), 1210 mg Potassium Chloride (KCl), 780 mg Sodium phosphate monobasic dihydrate ($NaH_2PO_4.2H_2O$), 5 mg Sodium sulphide nonahydrate ($Na_2S.9H_2O$), 1000 mg Urea ($CO(NH_2)_2$), 1000 mL deionised water.

The final pH of the artificial saliva was adjusted, using NaOH, to pH 6.4 ($\pm$ 0.15).

The positive and negative controls (Groups 3 and 4) were prepared with deionised water.

The pH of each solution was adjusted into the pH range of pH 6 to 6.5, using sodium hydroxide or dilute hydrochloric acid (Excluding the positive and negative control, which did not have the pH adjusted).

All pH measurements were taken with a calibrated pH electrode (Serial Number: VT1 8048) and an Accumet XI,250 ion meter (Asset Number: UK01200-LAB-15-002).

3.2.2 Block preparation

Approximately 130 enamel blocks (measuring 4mm $\times$ 4mm) were sectioned from bovine incisors and were lapped plano-parallel (both sides) using a Logitech PM5 lapping machine (Asset number: 4F008). The enamel surfaces of the samples were machine-polished with 1-micron and 0.3-micron aluminium oxide slurries, using a MetPrep Saphir 550 Polisher (Asset Number: UK01200-1010015-114). One corner of each block was removed to facilitate consistent orientation of the samples on the surface microhardness machine. All blocks were stored in individual Sterilins, on a layer of tissue paper dampened with 0.1% thymol. An additional set of 30 blocks was prepared according to the steps above, to enable top-up groups to be run, which replaced the data for the treatments that were initially not prepared in accordance with the analytical protocol.

3.2.3 Surface Microhardness (SMH) Measurements

The surface microhardness of each block was measured at the following time points:

- At baseline.
- After the demineralisation treatment.
- After overnight treatment.
- After the acid challenge.

The surface microhardness of the enamel samples was measured using a Buehler MicroMet 5103 (Asset Number: 4F101) and OmniMet MHT software.

The Intertek CRS method for measuring the surface microhardness of the samples at each time point has been provided below:

- Five Knoop indents
- 50-gram load force
- 10-second dwell time
- X50 objective

The schematic diagram in Figure 2, shows the approximate location of the indents. Spacings were approximate and dependent on the size of the indents and the avoidance of surface scratches and other imperfections.

Following the acid challenge, the indents were too large to accurately view on the x50 objective lens of the Buehler MicroMet 5103.

As a result, the surface microhardness analysis of the post-acid challenge samples was performed using a Wilson, Tukon 1202 SMH machine and an x10 objective lens. All other surface microhardness settings remained the same.

3.2.4 Removal of Outliers and Stratification

Specimens with "outlier" baseline SMH values were removed from the study. 110 blocks were stratified into 11 treatment groups (n=10) to create groups with similar mean baseline SMH values.

3.2.5 Enamel Block Demineralisation

A demineralisation solution was prepared that contained: 50 mM Acetic acid, 2.2 mM Calcium nitrate, 2.2 mM Potassium phosphate monobasic, 0.1 ppm Sodium fluoride. The pH of the solution was adjusted to pH 4.5. All pH measurements were taken with a calibrated pH electrode (Serial Number: VT1 8048) and an Accumet XI,250 ion meter (Asset Number: UK01200-LAB-15-002.

The enamel blocks were demineralised by placing the samples into a Sterilin containing 8ml of demineralisation solution per sample. All demineralisation exposures were performed for 60 minutes, in an oven set to 37°C.

Following the removal of the samples from the demineralisation solution, each enamel block was rinsed with deionised water for 1-2 minutes and returned to its original container. The post demineralisation surface microhardness of the samples was then remeasured.

3.3 Treatment

3.3.1 Test Product Treatment

All treatments were performed within 24 hours of the initial demineralisation of the samples being performed.

The enamel samples were placed into their assigned test solutions for 16 hours at 37°C, whilst being agitated with a magnetic stirrer. Aquasil Putty was used to attach the 10 enamel blocks in each treatment group to a sample holder with the enamel surfaces of the samples exposed.

Following the removal of the samples from the test product, each enamel block was rinsed with deionised water for 1-2 minutes and sonicated for 10 minutes to remove any loose debris. The enamel samples were then returned to their original containers.

3.3.2 Acid Challenge

All citric acid exposures were performed within 24 hours of the 16 treatments.

All acid exposures were performed by immersing the samples in 8ml of citric acid solution for 1-hour (37°C). Following exposure, the samples were rinsed with deionised water for 1-2 minutes.

3.4 Results and discussion

3.4.1 %SMHR Data

The mean %SMHR values achieved by the test products after a single treatment are shown in Table 8 and presented graphically in Figure 3.

Table 8. Post-Treatment Mean %SMHR

| Treatment | Mean Percentage Surface Microhardness Recovery (%SMHR) | StDev |
|---|---|---|
| Chitosan Hydrochloride 0.25% | 1.19 ($p < 0.033$) | 3.31 |
| Chitosan Hydrochloride 0.5% | 3.4 ($p < 0.05$) | 4.26 |
| Fluoride (Positive control) | 3.74 ($p < 0.016$) | 6.87 |
| Deionized water (Negative control) | -3.90 ($p < 0.05$) | 5.50 |
| Chitosan 0.5% | -1.2 ($p < 0.05$) | 3.27 |

3.4.2 %RER Data

The mean %RER values achieved by the test products after a single treatment are shown in Table 9 and presented graphically in Figure 4.

Table 9. %RER values after acid challenge

| Ingredient | %RER | StDev |
|---|---|---|
| Chitosan 0.5% | -50.99 ($p < 0.05$) | 30.01 |
| Chitosan hydrochloride 0.5% | -30.67 ($p < 0.05$) | 16.34 |
| Chitosan hydrochloride 0.25% | -41.9 ($p < 0.05$) | 11.8 |
| 1450 ppm Fluoride | -44.44 ($p < 0.05$) | 15.89 |
| Deionised Water | -91.25 ($p < 0.05$) | 30.01 |

Conclusion: Chitosan hydrochloride at both concentrations (0.25% and 0.5%) demonstrated a statistically significant increase in the Mean Percentage Surface Microhardness Recovery (%SMHR), indicating its potential effectiveness in enamel remineralization, with 0.5% concentration showing greater efficacy. Considering RER study these results, considering that all enamel blocks were exposed to acid for one hour after being treated with the solutions, suggest that chitosan hydrochloride at 0.25% and 0.5% concentrations may provide protection against acid erosion as good as positive control Fluoride, but 0.5% Chitosan showed protection too, but not as good as Chitosan hydrochloride form.

**Experiment 4.** In Vitro Study to Compare the Anti-Plaque Efficacy of Chitosan hydrochloride versus Chitosan

4.1 Study design

This in vitro method compared the ability of 2 test products versus a positive and negative control to prevent plaque biofilms from growing onto the surface of the glass rods.

The control products selected for this study were Corsodyl (Positive control) and sterile water (Negative control). The test products that were assessed in this study were supplied by SkyLab AG and prepared at Intertek CRS laboratory. The study methodology has been summarised below.

Dental plaque was grown over 3 days by immersing the roughened glass rods into fresh, pooled human saliva containing 0.1% sucrose. During treatment days 2 and 3, plaque growth was also encouraged by periodically exposing the glass rods to a nutrient broth containing tryptone soy broth (TSB), saliva, and sucrose.

On treatment day 1, the roughened glass rods were treated with a single application of the assigned treatment.

On treatment days 2 and 3, the roughened glass rods were treated with two applications of the assigned treatment.

At the end of the 3 days, the plaque biofilms were harvested from the roughened glass rods and the level of plaque growing on the rods was quantified by dry weight (g). Lower dry weights were indicative of greater anti-plaque efficacy.

4.2 Study Preparation

The study preparation involved preparing the following items:
A substrate for the biofilm to grow on.

- Fresh pooled saliva containing 0.1% sucrose.
- A growth medium.
- Test product solutions.

4.2.1 Substrate Preparation

Glass rods, 6 mm in diameter and 10 cm in length were used as a substrate for plaque growth, with 4 rods assigned to each treatment group. The rods were roughened along two-thirds of their length using 400 grit paper. The rods were rinsed thoroughly with deionised water before being left to air dry.

The rods were sonicated to remove any loose debris and sterilised in 70 % isopropanol for 1 hour before being left to dry in a microbiological safety cabinet.

A 6 mm diameter hole was drilled in the centre of the container lids. The holders were used to hold the glass rods in place during the study and were sterilised in 70 % isopropanol for 1 hour and then left to dry in a microbiology safety cabinet.

4.2.2 Saliva Containing 0.1 % Sucrose

Informed consent was provided by Intertek CRS staff volunteers who provided stimulated saliva (using flavourless gum) into 30 mL containers. All anonymised saliva donations were recorded using a donor log, with fresh saliva collected and pooled during each day of the study. When a sufficient volume of saliva was collected, 0.1 % sucrose (w/w) was added.

4.2.3 Nutrient Broth

The nutrient broth containing 3 % tryptone soy broth and 10 % sucrose was made in advance at 3rd party facility and sterilised by autoclaving. Prior to performing the nutrient immersions, 6.5 % of fresh saliva (w/w) was added to the broth.

4.3 Treatments

The test products that were assessed in this study are shown in Table 10.

The positive control (Corsodyl, containing 0.2% chlorhexidine) and the negative control (Sterile water) were used as supplied by the manufacturers. For all individual treatments, 4 mL of the control products were used.

All test products were originally going to be prepared in a single batch, in the concentrations shown in Table 10. However, the treatments soon solidified to the bottom of the Falcon tubes and could not get back into the solution. As a result, these tubes were discarded, and fresh treatments were prepared when needed. For all individual treatments, 4 mL of the test products were used.

Treatments consisted of immersing the rods in their respective treatments for 2 minutes, then using a pipette to gently rinse the rods with 3.5 mL of sterile water.

Table 10. Treatment Groups

| Group | Product Description |
|---|---|
| 1 | Chitosan hydrochloride (0.25 %) |
| 3 | Chitosan (0.5%) |
| 4 | Positive Control - Corsodyl Original Mouthwash |
| 5 | Negative Control - Distilled Sterile Water |

The sterilised rods were inserted into the lids ready for treatment inside the microbiology safety cabinet. The rods and lids were used as one from this point onwards.

On the afternoon of day 1, the sterilised glass rods were pre-treated with 4 mL of their respective treatment. After 2 minutes, the rods were removed, rinsed with sterile distilled water, and then placed into 4 mL of saliva (containing 0.1 % sucrose). The tubes were placed in a rack and transferred to an incubator for approximately 18 hours at 37°C, whilst being agitated on a plate shaker set to 250 rpm.

On the morning of days 2 and 3, the rods were once again treated, before being placed into a nutrient broth and transferred back into the incubator and agitated. After 6 hours in the broth, the rods were treated again, transferred into saliva containing 0.1 % sucrose, and placed in the incubator for a further 18 hours.

On day 4, the Falcon tubes containing the glass rods were removed from the incubator and were used to determine the dry weights of plaque biofilms.

4.3.1 Dry Weight Analysis

The rods were removed from their Falcon tubes and allowed to air dry. Rods were then weighed on a 4 decimal place analytical balance. After re-hydration the biofilms were removed, and the rods were re-weighed.

The dry weight of biofilm was determined from the following equation:

$$Weight\ of\ biofilm\ (g) = Weight\ of\ rod\ and\ biofilm\ (g) - Weight\ of\ rod\ (g)$$

4.4 Results

The mean plaque dry biofilm weights that were achieved by each treatment are shown in Table 11 and presented graphically in Figure 5.

Table 11. Post Treatment Mean Plaque Dry Biofilm Weights (g)

| Treatment | Mean Plaque Dry Biofilm Weight (g) | StDev |
|---|---|---|
| Chitosan Hydrochloride (0.25 %) | 0.0020 ($p < 0.05$) | 0.0002 |
| Corsodyl Original Mouthwash | 0.0003 ($p < 0.05$) | 0.0003 |
| Distilled Sterile Water | 0.0028 ($p < 0.05$) | 0.0002 |
| Chitosan 0.5% | 0.0023 ($p < 0.05$) | 0.0002 |

Corsodyl was selected as the positive control in this test because of its well-established and effective antimicrobial efficacy. Distilled sterile water was selected as the negative control for this test because it has no known antimicrobial properties. Statistical analysis of the plaque dry weight data showed sterile water (Negative control) achieved a statistically significantly higher biofilm weight than other comparator products that were assessed in this study.

Chitosan hydrochloride at 0.25% was statistically more effective in reducing plaque biofilm formation compared to chitosan at 0.5%.

**Experiment 5**. In Vitro Study to Compare the Ability of a Chitosan Hydrochloride Versus a Negative Control (Deionised Water) to Adhere to the Surface of Enamel

5.1 Study Design

Twelve enamel blocks were sectioned from bovine teeth, lapped plano-parallel, and polished. The surfaces of all samples were assessed with a light microscope to ensure the surfaces of the samples were free of contaminants. Following the inspection of the samples, a light microscope was used to capture representative images of the samples before treatment.

Three enamel samples were randomly assigned to each treatment group and treated with a single 3 minute application of the test compound or the negative control. Following treatment, the enamel samples were dried and re-imaged using a light microscope.

The enamel samples in the dip-wash sub-group were immersed in deionised water three times, to assess the ability of the test compound or negative control to remain on the surface of the enamel samples. Following dip washing, the samples were air dried and re-imaged using a light microscope.

The enamel samples in the flow sub-group were exposed to a constant microfluidic flow of deionised water over the samples for 1, 5, and 30 minutes. The microfluidic flow treatment aimed to assess the ability of the test compound versus the negative control to remain on the surface of the enamel samples. Following each exposure timepoint, the samples were air dried and re-imaged using a light microscope.
The ability of the treatments to remain on the surfaces of the enamel samples was determined by a visual inspection of the light microscope images.

5.2 Test Products

Table 12. Test Products & Treatment Regime

| Treatment Group | Test Product | Rinse Applied |
|---|---|---|
| 1 | Chitosan Hydrochloride (0.2%) in deionised water. | x3 deionised water dip washes. |
| 2 | Chitosan Hydrochloride (0.2%) in deionised water. | 1, 5, & 30 minutes of microfluidic flow of deionised water over the samples. |
| 3 | Scientific Laboratory Supplies, Product Code: CHE3876<br>Batch: 892898<br>Exp: 25/10/2024 | x3 deionised water dip washes. |
| 4 | Scientific Laboratory Supplies, Product Code: CHE3876<br>Batch: 892898<br>Exp: 25/10/2024 | 1, 5, & 30 minutes of microfluidic flow of deionised water over the samples. |

5.3 Block Preparation

Twelve bovine enamel blocks (Measuring 6 mm × 6 mm) were sectioned from bovine incisors and were lapped plano-parallel (both sides) using a Logitech PM5 lapping machine. The enamel surfaces of the samples were hand-polished with 2500 grit silicon carbide paper.

5.3.1 Baseline Microscopy

All enamel samples were inspected using an Olympus BX53M microscope (x5 object lens), to ensure the surfaces of the enamel samples were free of contaminants. Following the surface checks, representative, pre-treatment images of the enamel samples were captured.

5.3.2 Test Product Preparation

A test product solution was prepared by weighing 0.1 grams of Chitosan hydrochloride and making up to 50 grams using deionised water. The pH of the solution was adjusted to pH 6 to 6.5 a dilute hydrochloric acid solution and a calibrated pH electrode.
The deionised water (Negative control) was used as supplied by the manufacturer.

5.4 Treatment

The test compound solution or the negative control were mixed prior to use. The enamel samples were placed into their assigned test product for 3 minutes (Room temperature, without agitation).
Following treatments, all enamel samples were dried for 10 minutes in an oven set to 37°C.

5.4.1 Post-Treatment Microscopy

An Olympus BX53M microscope (x5 object lens) was used to capture a post-treatment image of the samples after treatment with the test compound or deionised water.

5.4.2 Dip Wash Treatments

The enamel samples were held securely using tweezers and inserted into deionised water. Each dip wash treatment comprised of the enamel samples being subjected to a single upwards and downwards motion of the samples through 3 cm of deionised water.

5.4.3 Microfluidic Flow Treatments

The enamel samples were held on a microscope slide and custom jig was used to enable the deionised water to be flowed over the treated enamel samples (Figure 6).

An Elveflow OB 1 flow controller was used to flow deionised water (Via an Elveflow FS3 flow sensor) over the treated enamel samples, under a constant pressure of 30 mbar (Figure 7). The pressure of 30 mbar that was selected for this study produced a flow rate of approximately 0.3ml of deionised water per minute.

The microfluidic flow rate elected for this study was based upon a publication by Iorgulescu (2009) [41], which reported a whole unstimulated saliva flow rate, as approximately 0.3 to 0.4 ml per minute.

The microfluidic flow controller was used to flow deionised water over the samples for 1, 5, and 30 minutes. All microfluidic flow treatments were performed at room temperature, with the tip of the flow applicator held 3-4mm above the surface of the samples.

5.4.4 Post-Flow Exposure Microscopy Imaging

The enamel surface of each sample in the dip-washing groups were imaged using an Olympus BX53M microscope (x5 objective lens) after 3 dip wash treatments.

The enamel surface of each sample in the microfluidic flow groups were imaged after 1, 5 and 30 minutes of flow.

5.5 Results

Figure 8 provides a representative image of the enamel samples before the treatments were applied.

It can be seen from Figure 8, that the surface of the untreated enamel sample surface is free from deposits.

A representative image of the enamel treated with 0.2% Chitosan hydrochloride and deionised water are provided in Figure 9 and Figure 10.

The samples treated with 0.2% Chitosan Hydrochloride (Figure 9) contain visible deposits on the surfaces of the enamel samples after treatment, showing deposition of the test compound on the surfaces of the samples.

The enamel samples treated with deionised water show no visible traces of any deposition on the surfaces of the samples (Figure 10).

5.5.1 Dip Washing Subgroup Results

A microscopy image of the treated enamel samples subjected to three dip wash exposures is provided in Figure 11.

Following 3 dip wash cycles, the microscopy images of the enamel samples treated with 0.2% Chitosan Hydrochloride shows surface deposits remained on the surfaces of the enamel samples. The microscopy images show the test compound had sufficient adhesive properties to withstand 3 dip wash cycles with deionised water (Figure 11).

The enamel samples pre-treated with deionised water remain unchanged, with the microscopy images showing no surface deposits on the enamel samples after treatment or after the 3 dip wash cycles (Figure 11).

5.5.2 Saliva Flow Subgroup Results

A microscopy image of the treated enamel samples subjected to 1 minute of flow of deionised water over the surface of the samples is provided in Figure 12.

The microscopy images showed deposits of the 0.2% Chitosan hydrochloride test compound remained on the surfaces of the samples exposed to a 1 minute flow of deionised water. The data showed the test compound had sufficient adhesive properties to withstand 1 minute of deionised water being flowed over the samples at a rate of 0.3 ml per minute (Figure 12).

The enamel samples pre-treated with deionised water remain unchanged, as no surface deposits were detected on the enamel samples after treatment or after the 1 minute of flow exposure (Figure 12).

A microscopy image of the treated enamel samples subjected to 5 minutes of flow of deionised water over the surface of the samples is provided in Figure 13.

The microscopy images showed deposits of the 0.2% Chitosan hydrochloride test compound remained on the surfaces of the samples exposed to 5 minutes of flow of deionised water. The data showed the test compound had sufficient adhesive properties to withstand 5 minutes of deionised water being flowed over the samples at a rate of 0.3 ml per minute (Figure 13).

The enamel samples pre-treated with deionised water remain unchanged, as no surface deposits were detected on the enamel samples after treatment or after the 5 minute of flow exposure (Figure 13).

A microscopy image of the treated enamel samples subjected to 30 minutes of flow of deionised water over the surface of the samples is provided in Figure 14.

The microscopy images showed deposits of the 0.2% Chitosan hydrochloride test compound remained on the surfaces of the samples exposed to 30 minutes of flow of deionised water. The data showed the test compound had sufficient adhesive properties to withstand 30 minutes of deionised water being flowed over the samples at a rate of 0.3 ml per minute (Figure 14).

The enamel samples pre-treated with deionised water remain unchanged, as no surface deposits were detected on the enamel samples after treatment or after the 30 minute of flow exposure (Figure 14).

Conclusion: The 0.2% Chitosan hydrochloride compound showed adhesive properties versus deionised water, when evaluated under the test conditions described in this experiment.

Literature

1. N.J. Cochrane, F. Cai, N.L. Huq, M.F. Burrow, E.C. Reynolds. New approaches to enhanced remineralization of tooth enamel \\ J Dent Res, 89 (11) (2010), pp. 1187-1197.
2. J. Arends, J.M. Ten Cate. Tooth enamel remineralization \\ J Crys Growth, 53 (1) (1981), pp. 135-147.
3. J.D.B. Featherstone. Dental caries: a dynamic disease process \\ Aust Dent J, 53 (2008), pp. 286-291.
4. S. Shah. Statherin-role in biomimetic early caries management \\ Acta Scientific Dent Sci, 2 (6) (2018), pp. 57-60.
5. E.A. Martinez-Mier, D.B. Shone, C.M. Buckley, M. Ando, F. Lippert, A.E. Soto-Rojas. Relationship between enamel fluorosis severity and fluoride content \\ J Dent, 46 (2016), pp. 42-46.
6. F.V. Zohoori, A. Maguire. Are there good reasons for fluoride labelling of food and drink \\ Br Dent J, 224 (4) (2018), pp. 215-217.
7. I.A. Ball. The 'fluoride syndrome': occult caries? \\ Br Dent J, 160 (3) (1986), pp. 75-76.
8. N. Philip. State of the art enamel remineralization systems: the next frontier in caries management \\ Caries Res, 53 (2019), pp. 284-295.
9. G. Goobes, R. Goobes, W.J. Shaw, J.M. Gibson, J.R. Long, V. Raghunathan, O. Schueler-Furman, J.M. Popham, D. Baker, C.T. Campbell, P.S. Stayton, G.P. Drobny. The structure, dynamics, and energetics of protein adsorption - lessons learned from adsorption of statherin to hydroxyapatite \\ Magn Reson Chem, 45 (2007), pp. S32-S47.
10. C. Li, J. Wang, Y. Wang, H. Gao, G. Wei, Y. Huang, H. Yu, Y. Gan, Y. Wang, L. Mei, H. Chen, H. Hu, Z. Zhang, Y. Jin. Recent progress in drug delivery \\ Acta Pharm Sin B, 9 (6) (2019), pp. 1145-1162.
11. J. Liang, X. Peng, X. Zhou, J. Zou, L. Cheng. Emerging applications of drug delivery systems in oral infectious diseases prevention and treatment \\ Molecules, 25 (3) (2020), pp. 516-545.
12. M. Pandya, T.G.H. Diekwisch. Enamel biomimetics-Fiction or future of dentistry \\ Int J Oral Sci, 11 (2019), p. 8.
13. D.K. Yarbrough, E. Hagerman, R. Eckert, J. He, H. Choi, N. Cao, K. Le, J. Hedger, F. Qi, M. Anderson, B. Rutherford, B. Wu, S. Tetradis, W. Shi. Specific binding and mineralization of calcified surfaces by small peptides \\ Calcif Tissue Int, 86 (1) (2010), pp. 58-66.
14. A. George, L. Bannon, B. Sabsay, J.W. Dillon, J. Malone, A. Veis, N.A. Jenkins, D.J. Gilbert, N.G. Copeland. The carboxyl-terminal domain of phosphophoryn contains unique extended triplet amino acid repeat sequences forming ordered carboxyl-phosphate interaction ridges that may be essential in the biomineralization process \\ J Biol Chem, 271 (51) (1996), pp. 32869-32873.
15. Y. Yang, X.P. Lv, W. Shi, J.Y. Li, D.X. Li, X.D. Zhou, L.L. Zhang. 8DSS-promoted remineralization of initial enamel caries in-vitro \\ J Dent Res, 93 (5) (2014), pp. 520-524.
16. Y.P. Zhang, C.S. Din, S. Miller, S.A. Nathoo, A. Gaffar. Intra-oral remineralization of enamel with a MFP/DCPD and MFP/silica dentifrice using surface microhardness \\ J Clin Dent, 6 (2) (1995), pp. 148-153.
17. R.J. Sullivan, A. Charig, J. Blake-Haskins, Y.P. Zhang, S.M. Miller, M. Strannick, A. Gaffar, H.C. Margolis. In-vivo detection of calcium from dicalcium phosphate dihydrate dentifrices in demineralized human enamel and plaque \\ Adv Dent Res, 11 (4) (1997), pp. 380-387.
18. A.K. Burwell, L.J. Litkowski, D.C. Greenspan. Calcium sodium phosphosilicate (NovaMin®): remineralization potential \\ Adv Dent Res, 21 (1) (2009), pp. 35-39.
19. D.M. Da Camara, J.P. Pessan, T.M. Francati, J.A.S. Souza, M. Danelon, A.C.B. Delbem. Fluoride tooth paste supplemented with sodium hexametaphosphate reduces enamel demineralization in-vitro \\ Clin Oral Investig, 20 (8) (2016), pp. 1981-1985.
20. E.M. Takeshita, M. Danelon, L.P. Castro, R.F. Cunha, A.C.B. Delbem. Remineralizing potential of a low fluoride tooth paste with sodium trimetaphosphate: an in-situ study \\ Caries Res, 50 (6) (2016), pp. 571-578.
21. A.C.S.F. Zaze, A.P. Dias, K.T. Sassaki, A.C.B. Delbem. The effects of low-fluoride tooth paste supplemented with calcium glycerophosphate on enamel demineralization \\ Clin Oral Investig, 18 (6) (2014), pp. 1619-1624.
22. I.R. Freire, J.P. Pessan, J.G. Amaral, C.C. Martinhon, R.F. Cunha, A.C.B. Delbem. Anticaries effect of low-fluoride dentifrices with phosphates in children: a randomized controlled trial \\ J Dent, 50 (2016), pp. 37-42.
23. E.M. Takeshita, R.A. Exterkate, A.C.B. Delbem, J.M. Ten Cate. Evaluation of different fluoride concentrations supplemented with trimetaphosphate on enamel de- and remineralization in-vitro \\ Caries Res, 45 (5) (2011), pp. 494-497.
24. Hu, D.; Ren, Q.; Li, Z.; Zhang, L. Chitosan-Based Biomimetically Mineralized Composite Materials in Human Hard Tissue Repair \\ Molecules 2020, 25, 4785.
25. Cicciù, M.; Fiorillo, L.; Cervino, G. Chitosan Use in Dentistry: A Systematic Review of Recent Clinical Studies \\ Mar. Drugs 2019, 17, 417.
26. Akshat Gupta, Vidya Rattan, Sachin Rai. Efficacy of Chitosan in promoting wound healing in extraction socket: A prospective study \\ Journal of Oral Biology and Craniofacial Research, Volume 9, Issue 1, 2019. Pages 91-95.
27. Harun Achmad, Yunita Feby Ramadhany. Effectiveness of Chitosan Tooth Paste from White Shrimp (Litopenaeusvannamei) to Reduce Number of Streptococcus Mutans in the Case of Early Childhood Caries \\ Journal of International Dental and Medical Research. Volume 10, Number 2 (2017). P. 358-363.
28. Kmiec M, Pighinelli L, Tedesco MF, et al. Chitosan-properties and applications in dentistry \\ Adv Tissue Eng Regen Med Open Access. 2017;2(4):205-211.
29. 7. Thatiana M. Stamford Arnaud, Benício de Barros Neto, Flamarion B. Diniz. Chitosan effect on dental enamel de-remineralization: An in vitro evaluation \\ Journal of Dentistry, Volume 38, Issue 11, 2010, Pages 848-852.
30. Fakhri, E., Eslami, H., Maroufi, P., Pakdel, F., Taghizadeh, S., Ganbarov, K., Kafil, H. S. (2020). Chitosan biomaterials application in dentistry \\ International Journal of Biological Macromolecules.
31. Shun-yi Yao, Si-peng Chen, Ruo-xun Wang, Kai Zhang, Xiao-xuan Lin, Sui Mai. Antibacterial activity and bonding performance of carboxymethyl chitosan-containing dental adhesive system \\ International Journal of Adhesion and Adhesives, Volume 119, 2022.
32. Cai, M.; Ratnayake, J.; Cathro, P.; Gould, M.; Ali, A. Investigation of a Novel Injectable Chitosan Oligosaccharide-Bovine Hydroxyapatite Hybrid Dental Biocomposite for the Purposes of Conservative Pulp Therapy \\ Nanomaterials 2022, 12, 3925.
33. Zahra Shariatinia. Carboxymethyl chitosan: Properties and biomedical applications \\ International Journal of Biological Macromolecules, Volume 120, Part B, 2018, Pages 1406-1419.
34. Kumar, M. N. V. R., Muzzarelli, R. A. A., Muzzarelli, C., Sashiwa, H., & Domb, A. J. (2004). Chitosan Chemistry and Pharmaceutical Perspectives \\ Chemical Reviews, 104(12), 6017-6084.
35. Dalia Y. Zaki, Engie M. Safwat, Shaymaa M. Nagi, Haidy N. Salem, Tamer M. Hamdy, Lamiaa M. Moharam, Mohammad L. Hassan, E.M.A. Hamzawy. A novel dental remineralizing blend of hydroxyethyl-cellulose and cellulose nanofibers oral film loaded with nepheline apatite glass: Preparation, characterization and in vitro evaluation of remineralizing effect \\ Carbohydrate Polymer Technologies and Applications, Volume 2, 2021.
36. Fatullayeva, S., Tagiyev, D., Zeynalov, N. et al. Recent advances of chitosan-based polymers in biomedical applications and environmental protection \\ J Polym Res 29, 259 (2022).
37. Hemmingsen, L.M.; Škalko-Basnet, N.; Joraholmen, M.W. The Expanded Role of Chitosan in Localized Antimicrobial Therapy \\ Mar. Drugs 2021, 19, 697.
38. Peeters, E. Comparison of multiple methods for quantification of microbial biofilms grown in microtiter plates / E. Peeters, H.J. Nelis, T. Coenye // Journal of Microbiological MethodS. - 2008. - V.72(2). - C. 157-165.
39. Sambrook, J. Molecular cloning: a laboratory manual [Text] / J. Sambrook, E. F. Fritsch, T. Maniatis // Cold Spring Harbor Laboratory PresS. - 1989.
40. I.F.F. Benzie, J.J. Strain. The ferric reducing ability of plasma: FRAP as a measure of "antioxidant power" the FRAP assay, Anal. Biochem., 239 (1996), pp. 70-76.
41. Iorgulescu G. Saliva between normal and pathological. Important factors in determining systemic and oral health. J Med Life. 2(3) (2009), pp:303-307.

## Claims

1. Use of chitosan hydrochloride as a tooth enamel remineralizing agent in a dental care and/or oral care product.

2. The use of claim 1, the use of said chitosan hydrochloride further comprising the use as a growth inhibitor of oral bacteria, oral plaque or oral biofilm and/or the use as an antioxidant.

3. Use of a composition comprising chitosan hydrochloride for non-medical and/or aesthetic use, wherein said use is tooth and/or oral cavity cleaning or teeth brushing for mitigating aesthetic teeth enamel destruction results or prevention of it, including cracks and chips, discoloration and/or yellow staining, indentations, rough edges, shiny spots, translucent or fractured teeth.

4. A composition comprising chitosan hydrochloride for use in the remineralization of tooth in a subject.

5. The composition for use of claim 4, the use further comprising preventing destruction of a subject's tooth enamel.

6. The composition for use of claim 4 or claim 5, the use of said chitosan hydrochloride further comprising the use as a growth inhibitor of oral bacteria, oral plaque or oral biofilm and/or the use as an antioxidant.

7. The composition for use of any of claims 4-6, wherein said subject is suffering from or at risk of fluorosis or fluoride intoxication.

8. The use of claim 2 or the composition for use of claim 6 or claim 7, wherein said oral bacteria are selected from the group consisting of: *Streptococcus mutans, Streptococcus sobrinus, Streptococcus sanguis* and *Streptococcus aureus.*

9. The use or the composition for use of any of the preceding claims, wherein said dental care and/or oral care product or said composition comprises 0.10-1.00 wt.% of said chitosan hydrochloride.

10. The use or the composition for use of claim 9, wherein said product or composition comprises said chitosan hydrochloride as per the following wt.% sub-ranges in said composition: 0.15 - 0.95, 0.20 - 0.90, 0.25 - 0.85, 0.30 - 0.80, 0.35 - 0.75, 0.40 - 0.70, 0.45 - 0.65, 0.50 - 0.60, and combinations thereof.

11. The use or composition for use of any of the preceding claims, wherein said product or composition does not comprise fluoride.

12. The use or composition for use of any of the preceding claims, wherein said product or composition is selected from the group consisting of: a mouthwash, a toothpaste, tooth gel or powder, an oral foam, an oral gel, an oral teeth stripe or tooth cover, chewing gum.

13. The use or composition for use of any of the preceding claims, wherein said product or composition is a formulation selected from the following: film, liquid, aerosol, suspension, solution, tincture, cream, paste, lotion, ointment, gel, balsam, foam, powder, granulate.

14. The use or composition for use of any of the preceding claims, wherein the pH of said product or composition is above 6.0 and preferably below 11, preferably in the range of 6.0 to 7.5, further preferably in the range of 6.3-6.8.

15. The use or composition for use of any of the preceding claims, wherein said chitosan hydrochloride has the CAS number 70694-72-3.

Graphic of a visual comparison between MIC and MBIC

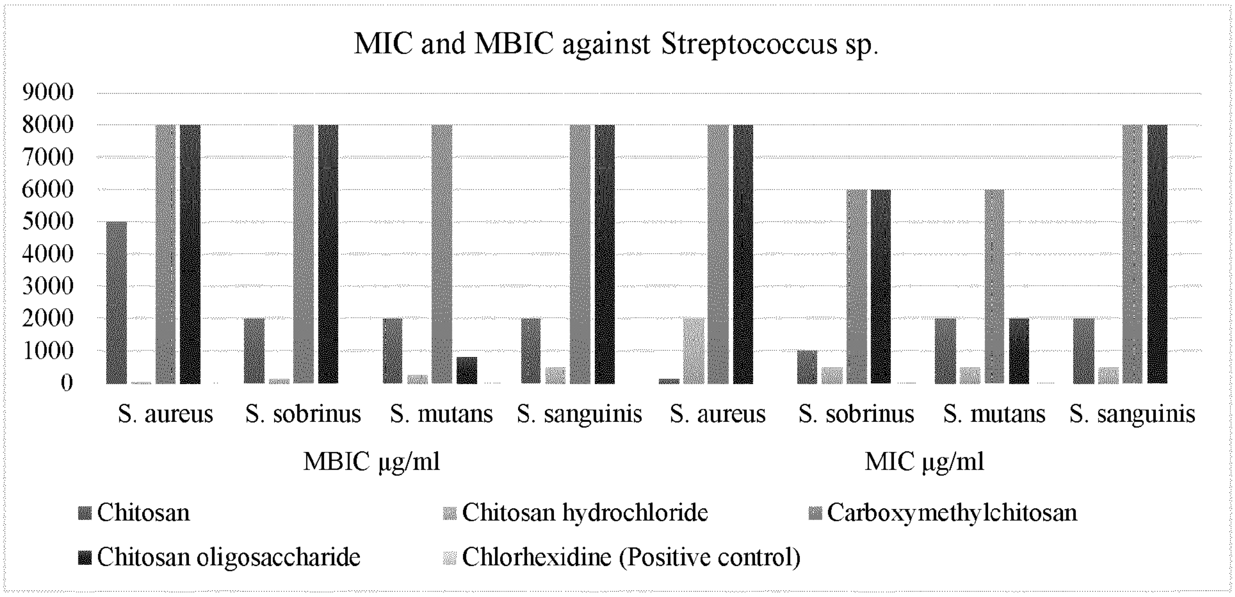


Fig. 1

Schematic Representation of SMH Indent Placement

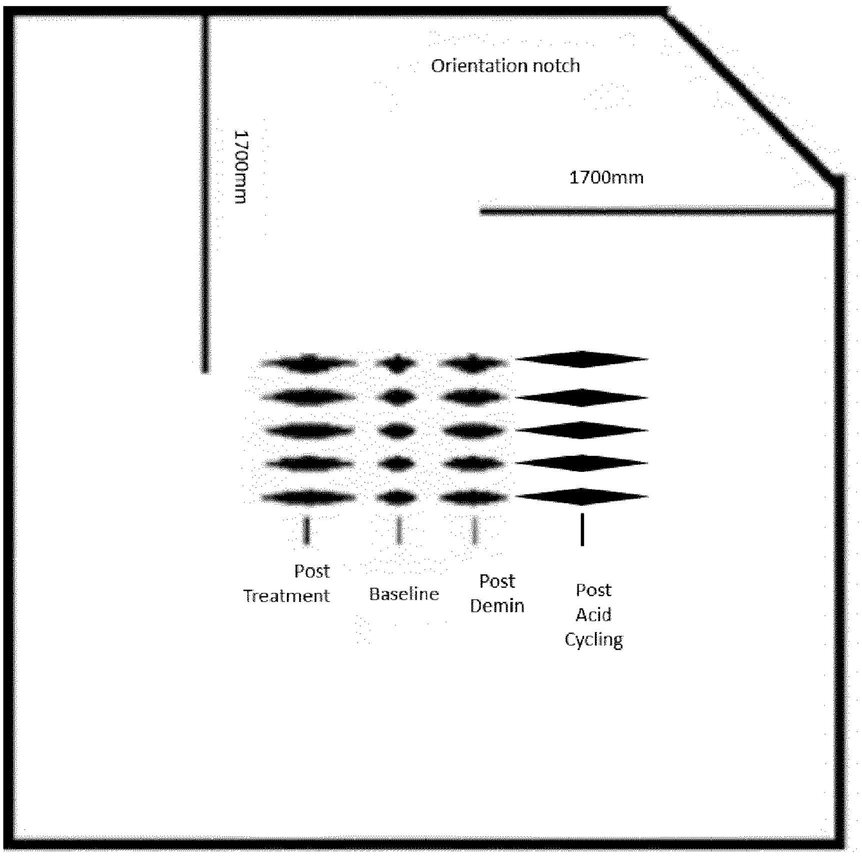


Fig.2.

The mean %SMHR values achieved by the test products

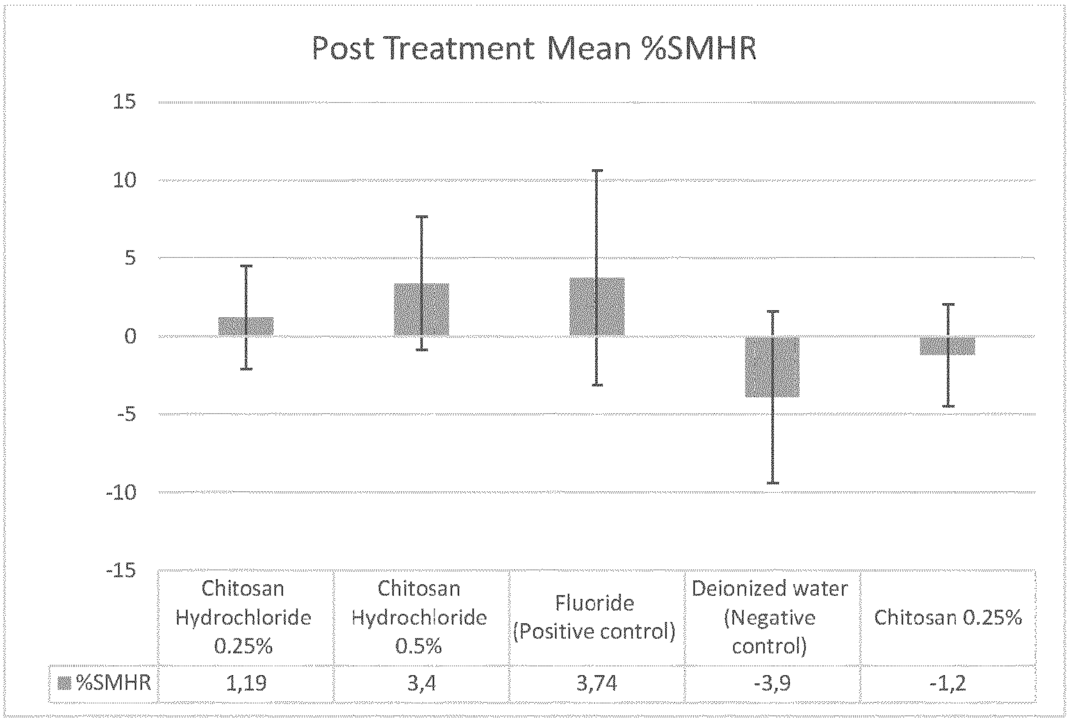


Fig. 3

%RER graphic after acid challenge

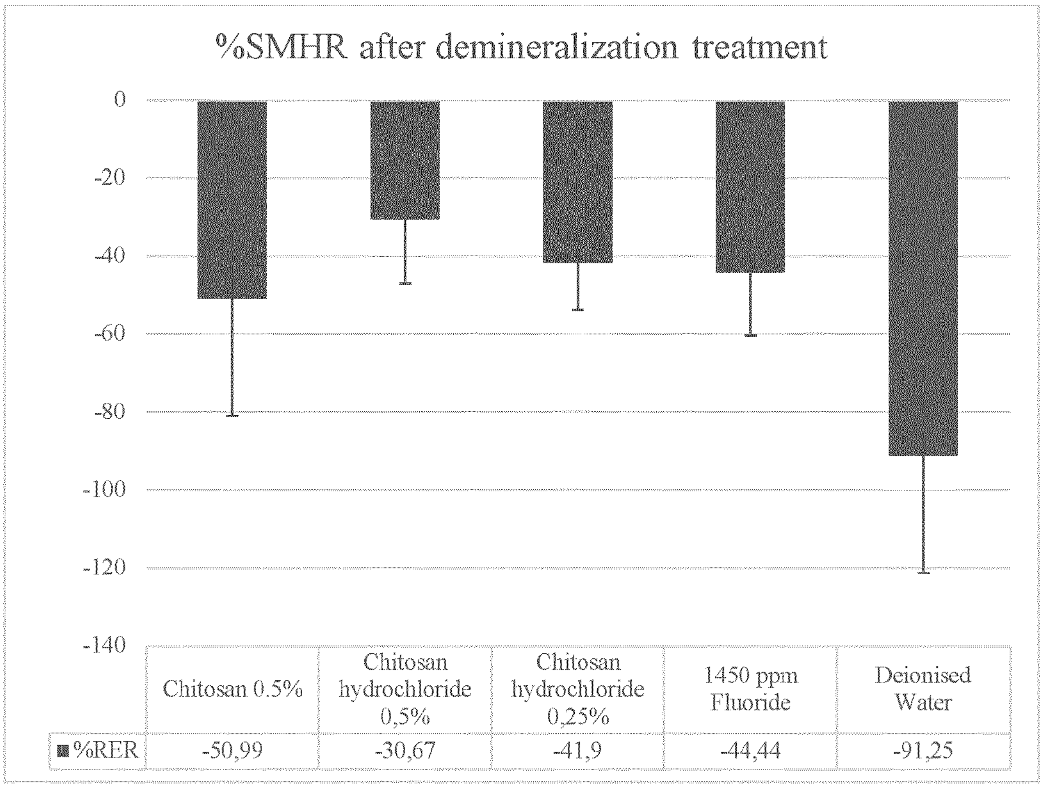


Fig.4

Post Treatment Mean Plaque Dry Biofilm Weights (g)

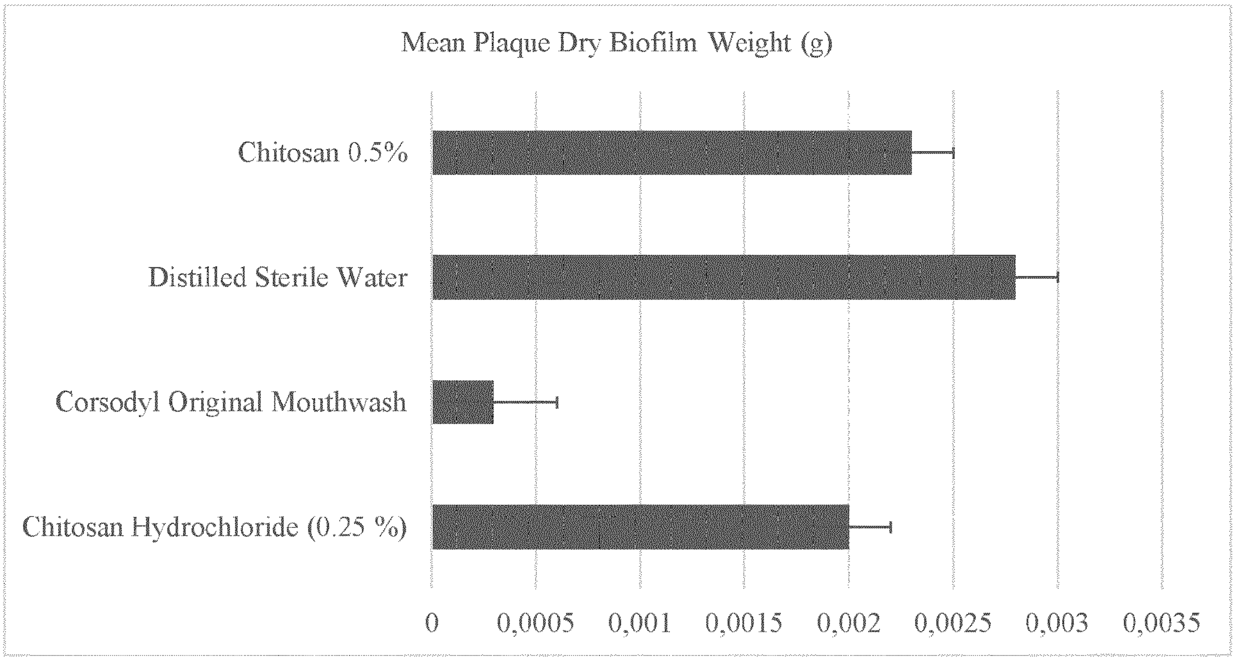


Fig. 5

Custom Jig Setup

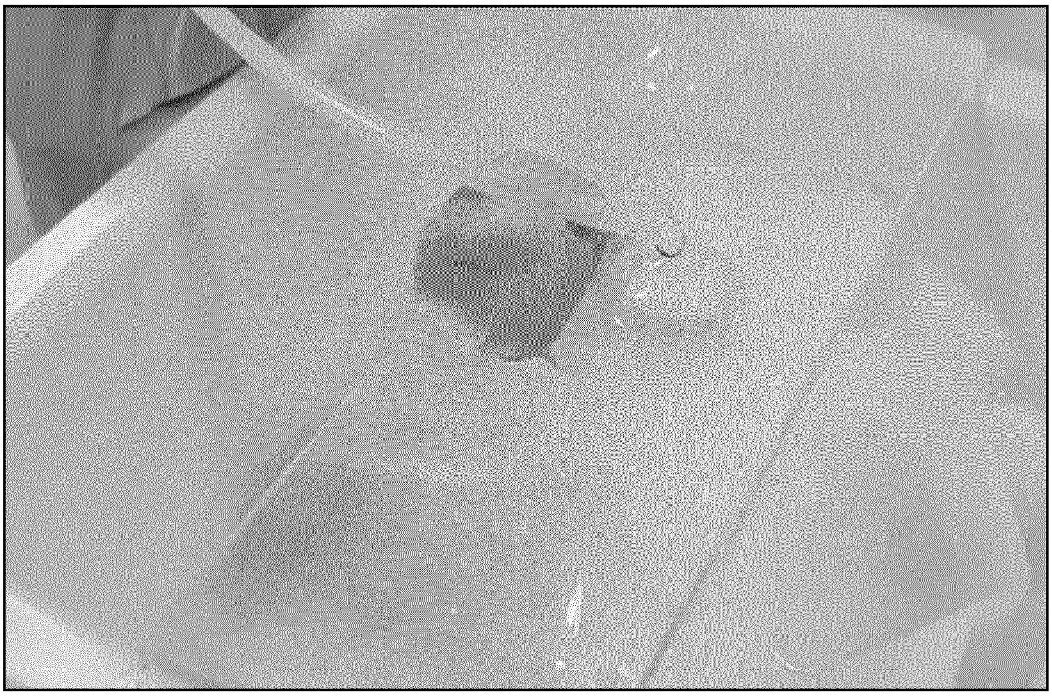

Fig. 6

The Elveflow system and salivary flow setup

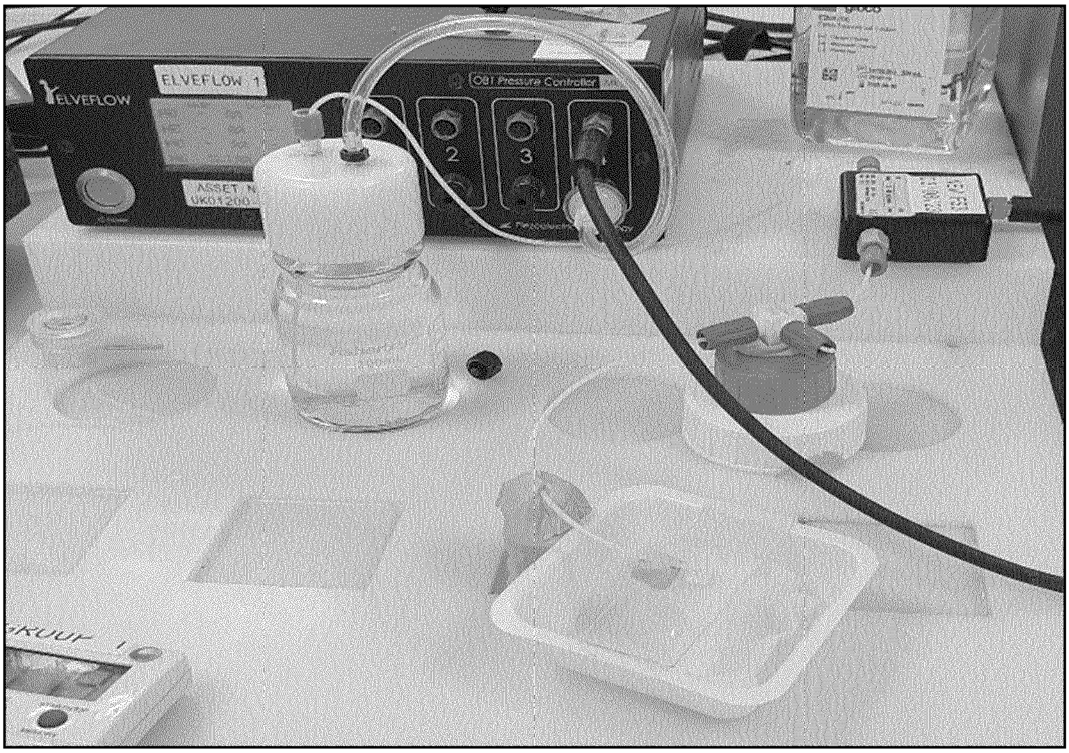


Fig. 7

Representative Pre-Treatment Image of an Enamel Sample

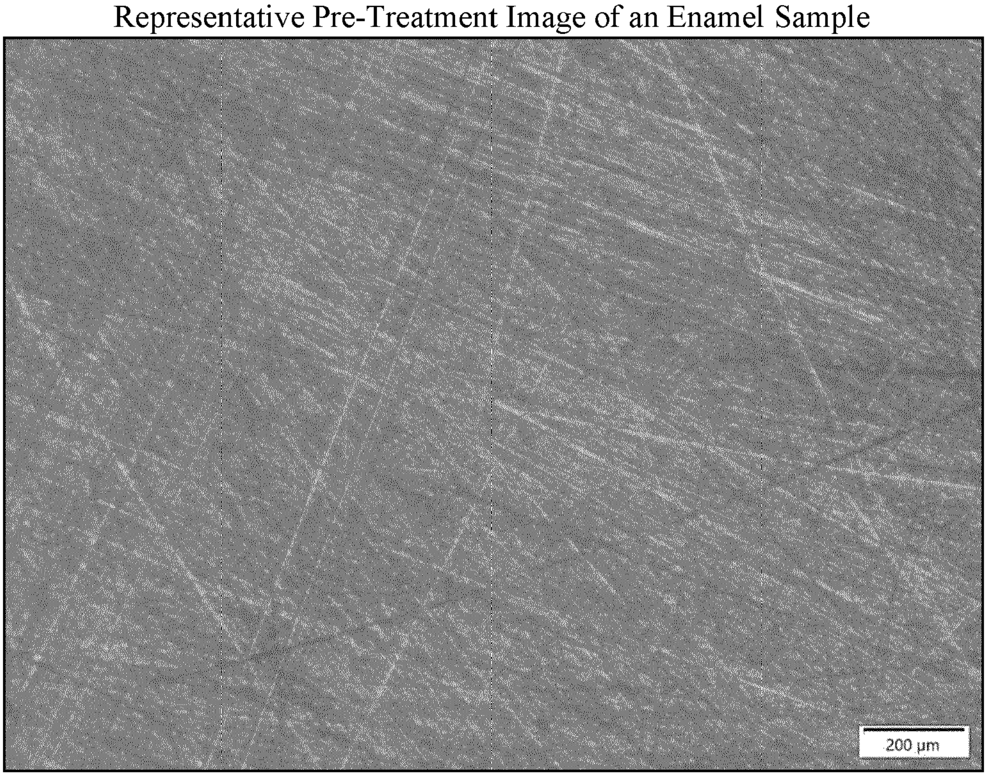


Fig. 8

Representative Image of an Enamel Sample Treated with 0.2% Chitosan hydrochloride

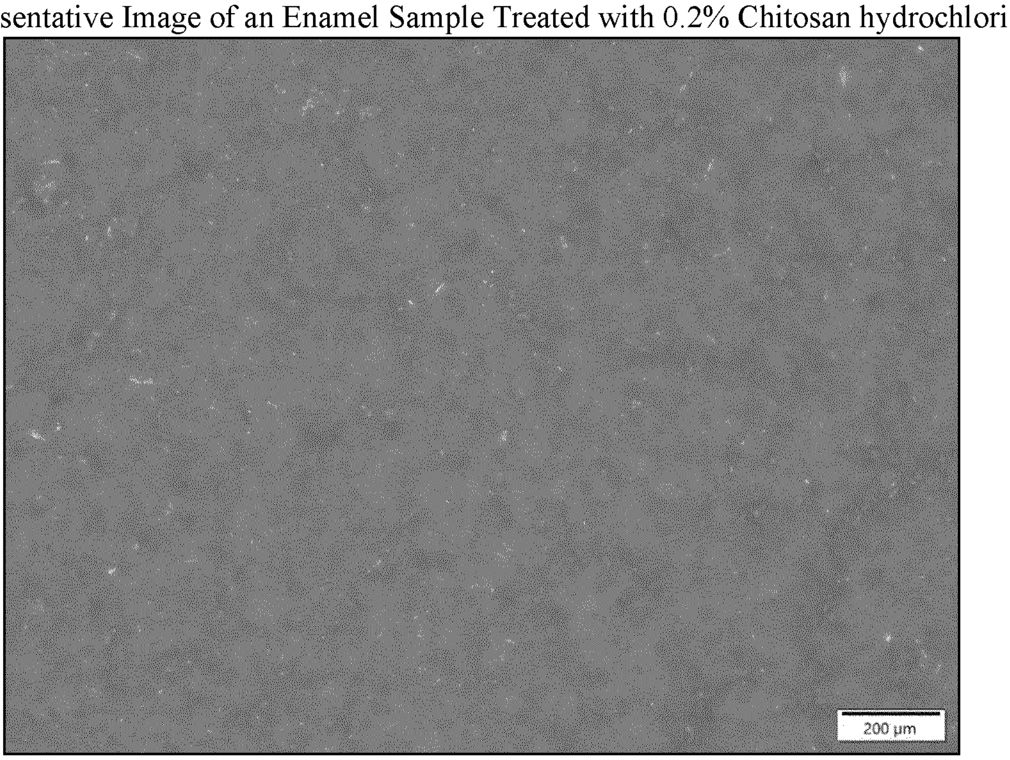


Fig. 9

Representative Image of an Enamel Sample Treated with Deionised Water

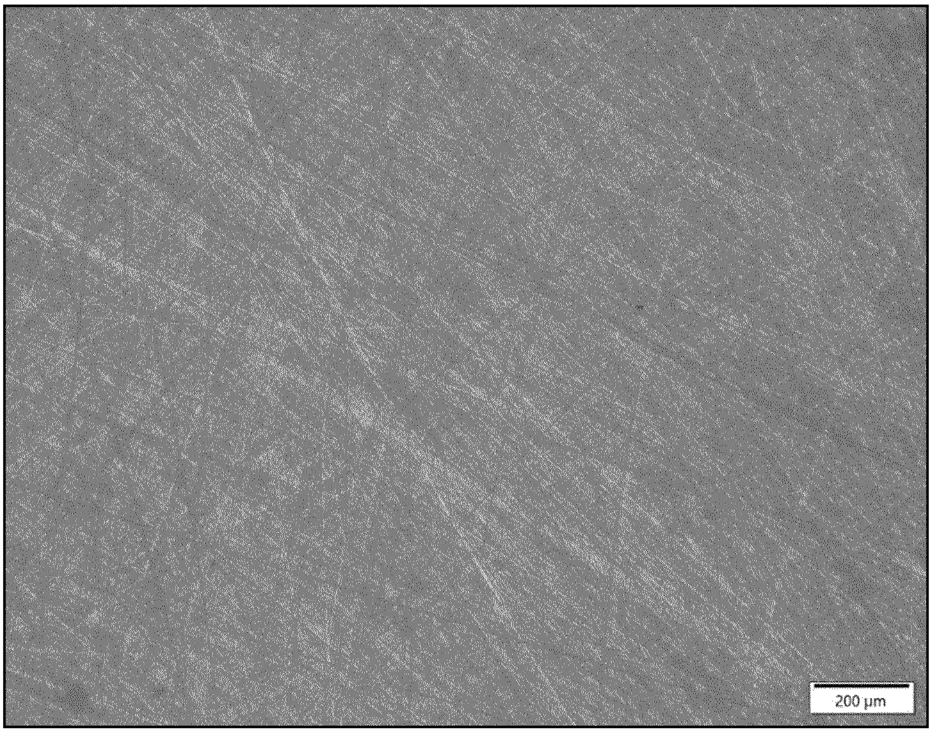


Fig. 10

Post Dip Wash Images of the Treated Enamel Samples

Sample 103 (Chitosan Hydrochloride)

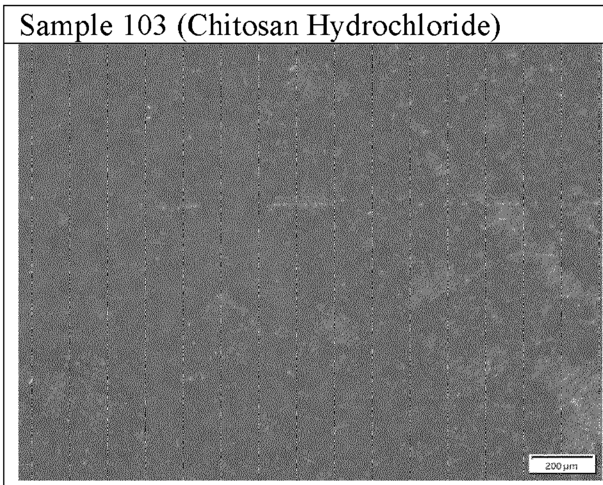


Sample 257 (Chitosan Hydrochloride)

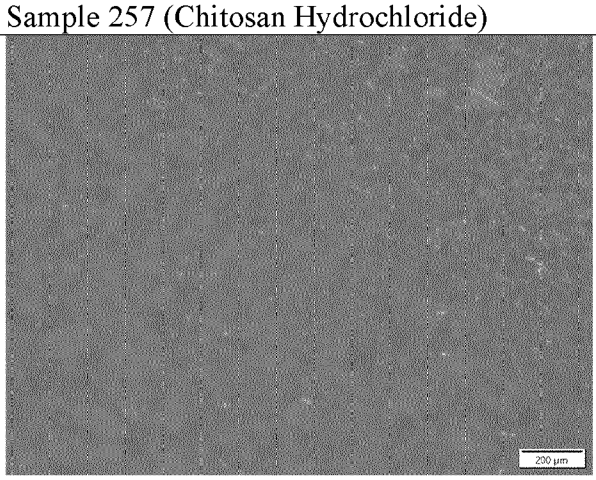


Sample 769 (Chitosan Hydrochloride)

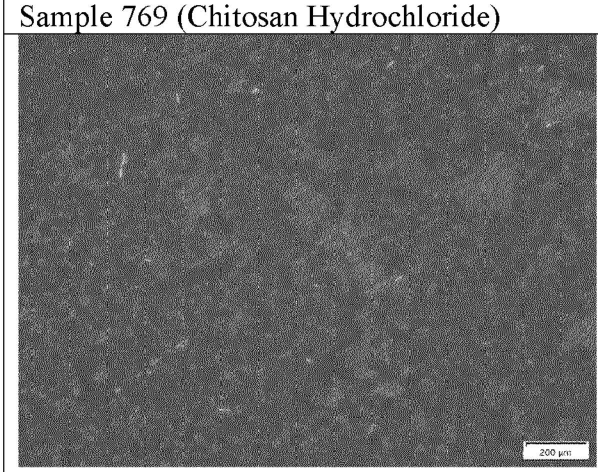


Sample 752 (Deionised Water)

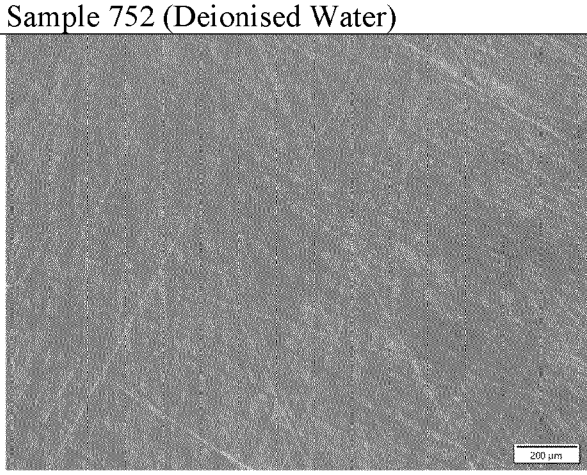


Sample 249 (Deionised Water)

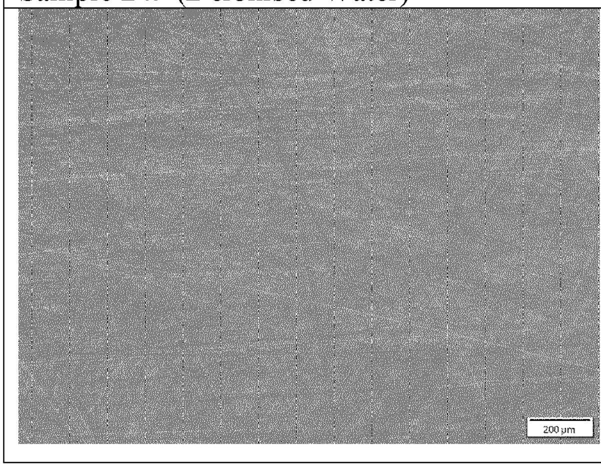


Sample 259 (Deionised Water)

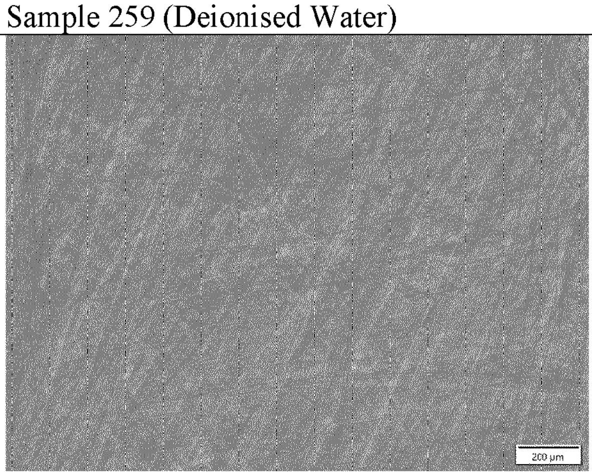


Fig. 11

Post 1 Minute of Flow Images

| Sample 28 (Chitosan hydrochloride) | Sample 770 (Chitosan hydrochloride) |
|---|---|
| 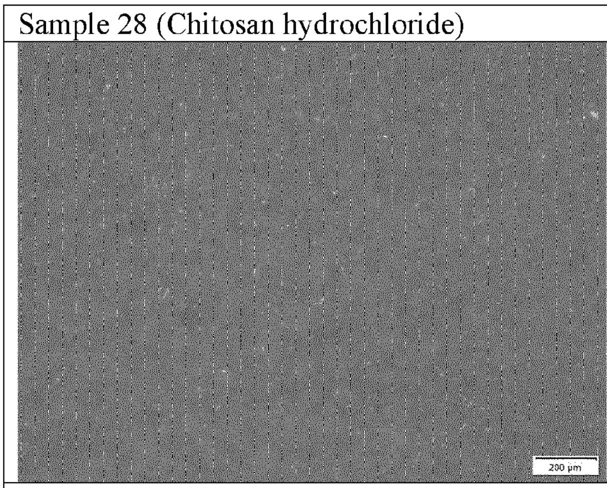 | 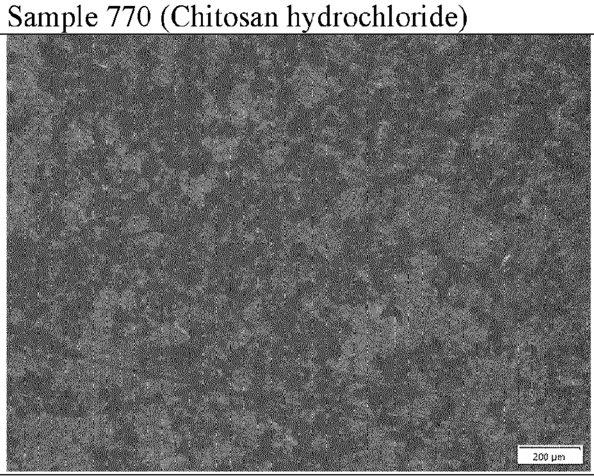 |
| Sample 793 (Chitosan hydrochloride) | Sample 294 (Deionised Water) |
| 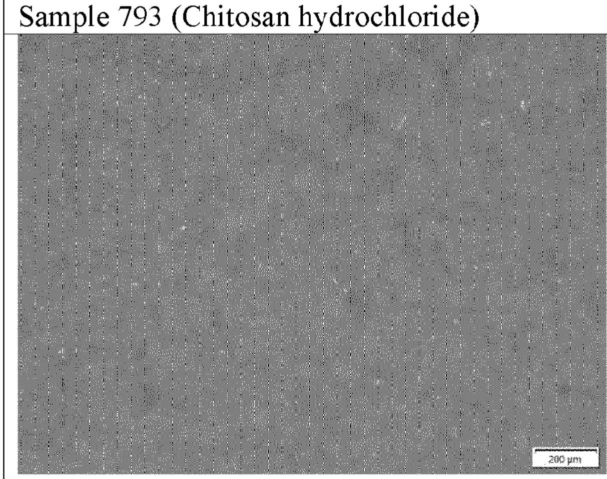 | 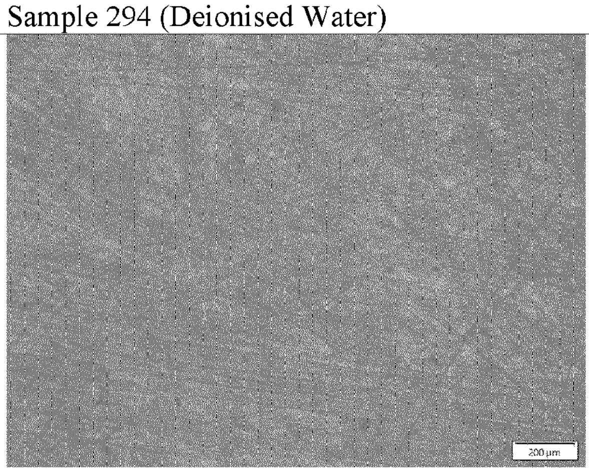 |
| Sample 2 (Deionised Water) | Sample 260 (Deionised Water) |
| 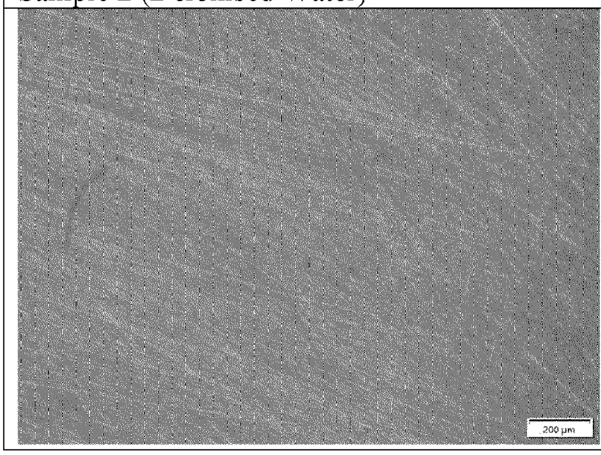 | 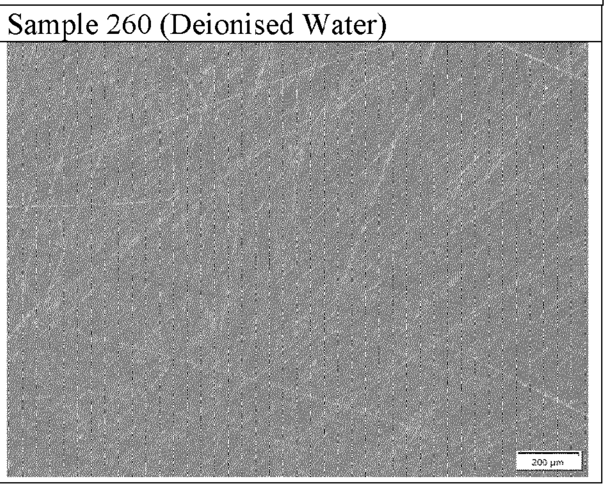 |

Fig. 12

Post 5 Minute of Flow Images

Sample 28 (Chitosan hydrochloride)

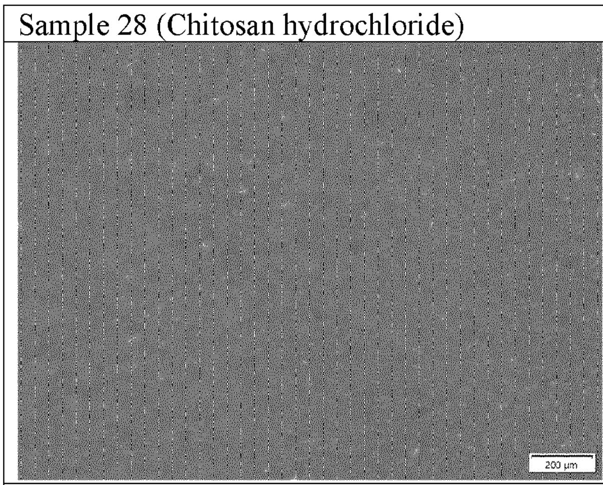


Sample 770 (Chitosan hydrochloride)

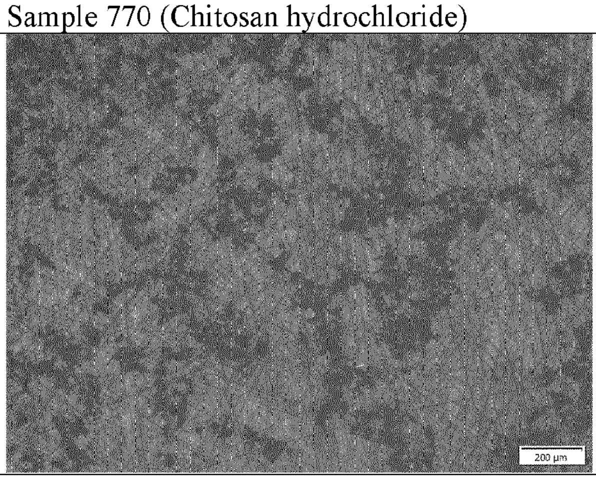


Sample 793 (Chitosan hydrochloride)

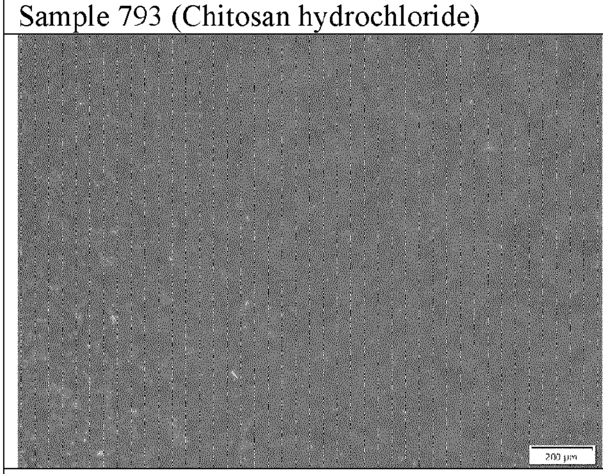


Sample 294 (Deionised Water)

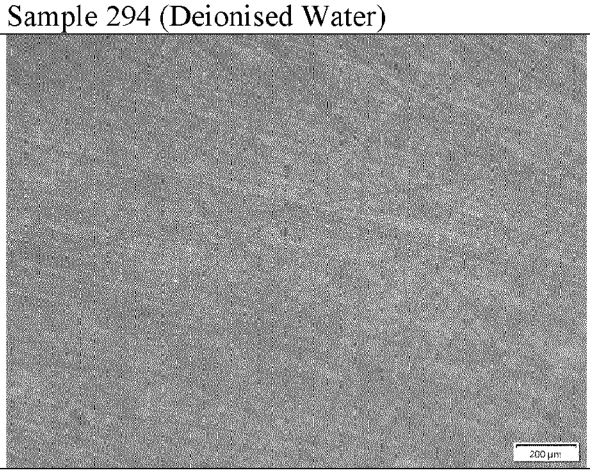


Sample 2 (Deionised Water)

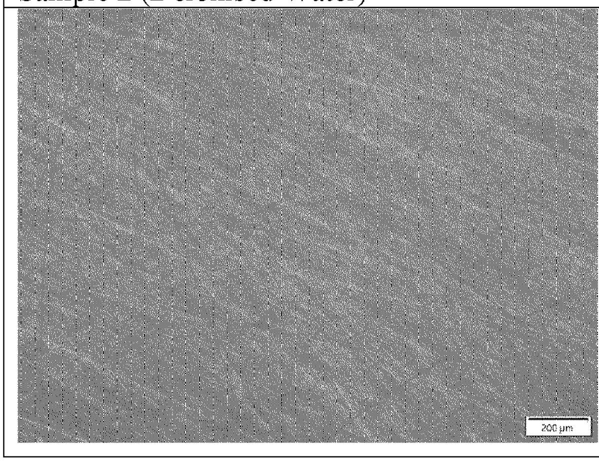


Sample 260 (Deionised Water)

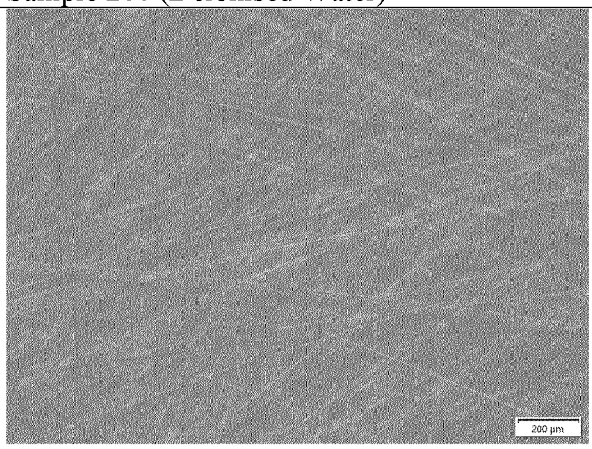


Fig. 13

Post 30 Minutes of Flow Images

| Sample 28 (Chitosan hydrochloride) | Sample 770 (Chitosan hydrochloride) |
|---|---|
| 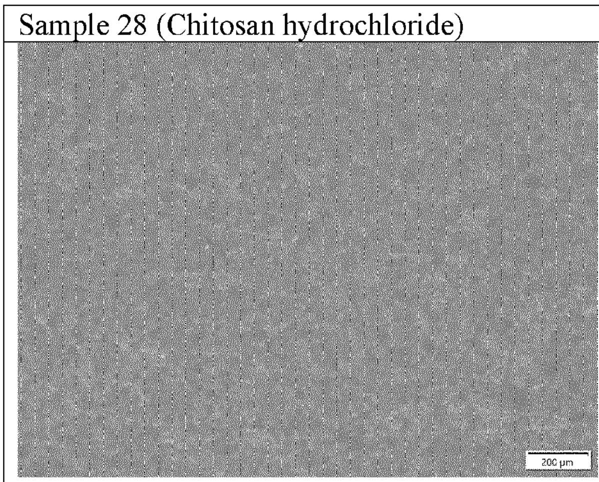 | 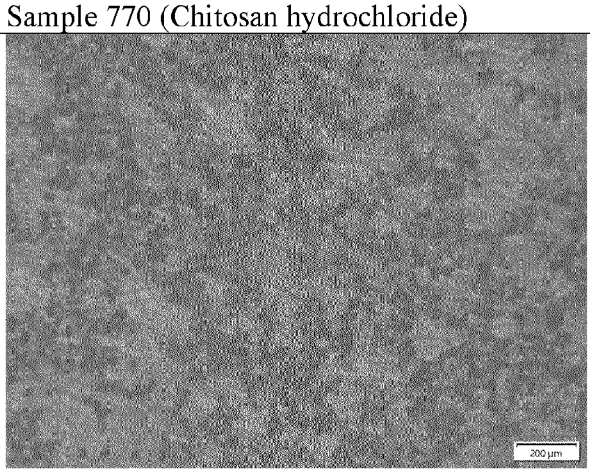 |
| Sample 793 (Chitosan hydrochloride) | Sample 294 (Chitosan hydrochloride) |
| 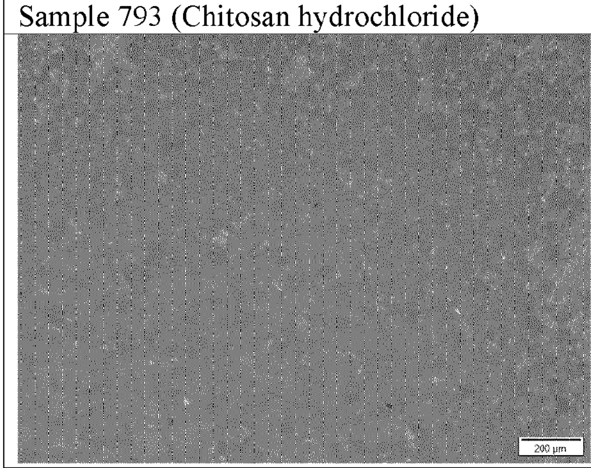 | 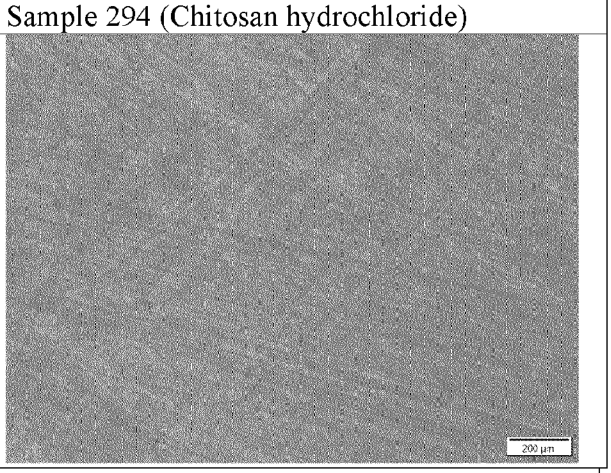 |
| Sample 2 (Deionised Water) | Sample 260 (Deionised Water) |
| 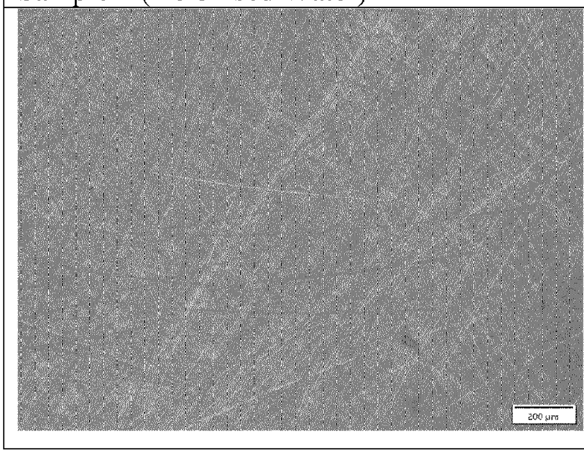 | 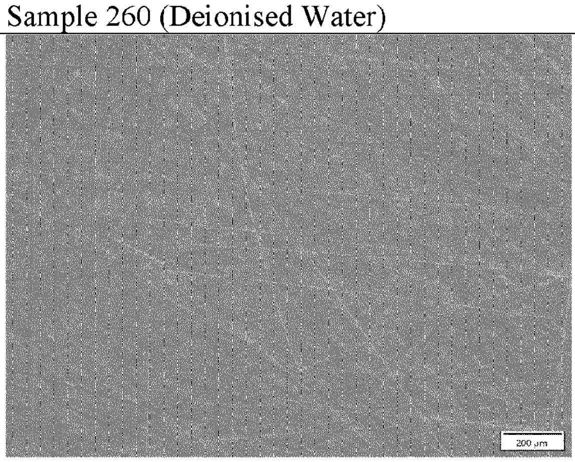 |

Fig. 14

Europäisches Patentamt
European Patent Office
Office européen des brevets

# EUROPEAN SEARCH REPORT

Application Number
EP 24 20 2082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 117 771 135 A (GUANGZHOU HOUDY GROUP CO LTD) 29 March 2024 (2024-03-29)<br>* the whole document *<br>* examples 1-10 * | 1-15 | INV.<br>A61K8/73<br>A61K31/722<br>A61P1/02<br>A61Q11/00 |
| X | RU 2 328 267 C1 (VSEROSSIJSKIJ NI I TI BIOLOG P [RU]; OOO PVE [RU])<br>10 July 2008 (2008-07-10)<br>* the whole document *<br>* examples 1-4 * | 1-7,9,<br>10,12,<br>13,15 | |
| X | KR 2020 0022837 A (D NATURE CO LTD [KR])<br>4 March 2020 (2020-03-04)<br>* the whole document *<br>* paragraphs [0055], [0058], [0065]; claims 1-6; examples 1-4 * | 1-3,5-7,<br>9-13,15 | |
| X | US 2019/060207 A1 (MORADIAN-OLDAK JANET [US] ET AL) 28 February 2019 (2019-02-28)<br>* the whole document *<br>* paragraph [0058]; claims 1-9 * | 1,3,4,<br>9-15 | TECHNICAL FIELDS SEARCHED (IPC) |
| X | JP H07 72123 B2 (LION CORP)<br>2 August 1995 (1995-08-02)<br>* the whole document *<br>* claim 1; table 2 * | 1-3,8-15 | A61K<br>A61P |
| X | JP H07 157434 A (TOYO SUISAN KAISHA)<br>20 June 1995 (1995-06-20)<br>* the whole document *<br>* paragraphs [0020] - [0028]; claims 1,2 * | 1-3,8-15 | |
| X | JP 2004 256557 A (UNIV NAGASAKI)<br>16 September 2004 (2004-09-16)<br>* the whole document *<br>* claims 1-6 * | 1,2,8-15 | |

-/--

The present search report has been drawn up for all claims

2

| Place of search | Date of completion of the search | Examiner |
|---|---|---|
| The Hague | 17 March 2025 | Jakobs, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 20 2082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2007 314505 A (UNIV NAGASAKI; LOTTE CO LTD) 6 December 2007 (2007-12-06) * the whole document * * paragraphs [0008] - [0013], [0021], [0034] * | 1-3,8-15 | |
| X | CN 107 280 978 A (HEHUA BIOTECHNOLOGY HANGZHOU CO LTD) 24 October 2017 (2017-10-24) * the whole document * * claims 1-20; examples 1-10 * | 1-3,8-15 | |
| X | JP 2004 091413 A (UNIV NAGASAKI) 25 March 2004 (2004-03-25) * the whole document * * claims 1-5 * | 1-3,8-15 | |
| A | NASAJ MONA ET AL: "Factors influencing the antimicrobial mechanism of chitosan action and its derivatives: A review", INTERNATIONAL JOURNAL OF BIOLOGICAL MACROMOLECULES, ELSEVIER BV, NL, vol. 277, 30 July 2024 (2024-07-30), XP087588694, ISSN: 0141-8130, DOI: 10.1016/J.IJBIOMAC.2024.134321 [retrieved on 2024-07-30] * the whole document * * table 4 * | 1-15 | TECHNICAL FIELDS SEARCHED (IPC) |
| A | CN 112 891 227 B (STOMATOLOGICAL HOSPITAL TIANJIN MEDICAL UNIV) 8 November 2022 (2022-11-08) * the whole document * | 1-15 | |

The present search report has been drawn up for all claims

2

| Place of search | Date of completion of the search | Examiner |
|---|---|---|
| The Hague | 17 March 2025 | Jakobs, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 20 2082

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2025

| Patent document cited in search report | Publication date | Patent family member(s) | Publication date |
|---|---|---|---|
| CN 117771135 A | 29-03-2024 | NONE | |
| RU 2328267 C1 | 10-07-2008 | NONE | |
| KR 20200022837 A | 04-03-2020 | NONE | |
| US 2019060207 A1 | 28-02-2019 | US 2014186273 A1 | 03-07-2014 |
| | | US 2019060207 A1 | 28-02-2019 |
| JP H0772123 B2 | 02-08-1995 | JP H0772123 B2 | 02-08-1995 |
| | | JP S61151112 A | 09-07-1986 |
| JP H07157434 A | 20-06-1995 | JP 2637041 B2 | 06-08-1997 |
| | | JP H07157434 A | 20-06-1995 |
| JP 2004256557 A | 16-09-2004 | JP 3728513 B2 | 21-12-2005 |
| | | JP 2004256557 A | 16-09-2004 |
| JP 2007314505 A | 06-12-2007 | NONE | |
| CN 107280978 A | 24-10-2017 | NONE | |
| JP 2004091413 A | 25-03-2004 | NONE | |
| CN 112891227 B | 08-11-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2020330360 A1
- GB 2132889 A

### Non-patent literature cited in the description


- **N.J. COCHRANE ; F. CAI ; N.L. HUQ ; M.F. BURROW ; E.C. REYNOLDS**. New approaches to enhanced remineralization of tooth enamel. *J Dent Res*, 2010, vol. 89 (11), 1187-1197
- **J. ARENDS ; J.M. TEN CATE**. Tooth enamel remineralization. *J Crys Growth*, 1981, vol. 53 (1), 135-147
- **J.D.B. FEATHERSTONE**. Dental caries: a dynamic disease process. *Aust Dent J*, 2008, vol. 53, 286-291
- **S. SHAH**. Statherin-role in biomimetic early caries management. *Acta Scientific Dent Sci*, 2018, vol. 2 (6), 57-60
- **E.A. MARTINEZ-MIER ; D.B. SHONE ; C.M. BUCKLEY ; M. ANDO ; F. LIPPERT ; A.E. SOTO-ROJAS**. Relationship between enamel fluorosis severity and fluoride content. *J Dent*, 2016, vol. 46, 42-46
- **F.V. ZOHOORI ; A. MAGUIRE**. Are there good reasons for fluoride labelling of food and drink. *Br Dent J*, 2018, vol. 224 (4), 215-217
- **I.A. BALL**. The 'fluoride syndrome': occult caries?. *Br Dent J*, 1986, vol. 160 (3), 75-76
- **N. PHILIP**. State of the art enamel remineralization systems: the next frontier in caries management. *Caries Res*, 2019, vol. 53, 284-295
- **G. GOOBES ; R. GOOBES ; W.J. SHAW ; J.M. GIBSON ; J.R. LONG ; V. RAGHUNATHAN ; O. SCHUELER-FURMAN ; J.M. POPHAM ; D. BAKER ; C.T. CAMPBELL**. The structure, dynamics, and energetics of protein adsorption - lessons learned from adsorption of statherin to hydroxyapatite. *Magn Reson Chem*, 2007, vol. 45, S32-S47
- **C. LI ; J. WANG ; Y. WANG ; H. GAO ; G. WEI ; Y. HUANG ; H. YU ; Y. GAN ; Y. WANG ; L. MEI**. Recent progress in drug delivery. *Acta Pharm Sin B*, 2019, vol. 9 (6), 1145-1162
- **J. LIANG ; X. PENG ; X. ZHOU ; J. ZOU ; L. CHENG**. Emerging applications of drug delivery systems in oral infectious diseases prevention and treatment. *Molecules*, 2020, vol. 25 (3), 516-545
- **M. PANDYA ; T.G.H. DIEKWISCH**. Enamel biomimetics-Fiction or future of dentistry. *Int J Oral Sci*, 2019, vol. 11, 8
- **D.K. YARBROUGH ; E. HAGERMAN ; R. ECKERT ; J. HE ; H. CHOI ; N. CAO ; K. LE ; J. HEDGER ; F. QI ; M. ANDERSON**. Specific binding and mineralization of calcified surfaces by small peptides. *Calcif Tissue Int*, 2010, vol. 86 (1), 58-66
- **A. GEORGE ; L. BANNON ; B. SABSAY ; J.W. DILLON ; J. MALONE ; A. VEIS ; N.A. JENKINS ; D.J. GILBERT ; N.G. COPELAND**. The carboxyl-terminal domain of phosphophoryn contains unique extended triplet amino acid repeat sequences forming ordered carboxyl-phosphate interaction ridges that may be essential in the biomineralization process. *J Biol Chem*, 1996, vol. 271 (51), 32869-32873
- **Y. YANG ; X.P. LV ; W. SHI ; J.Y. LI ; D.X. LI ; X.D. ZHOU ; L.L. ZHANG**. 8DSS-promoted remineralization of initial enamel caries in-vitro. *J Dent Res*, 2014, vol. 93 (5), 520-524
- **Y.P. ZHANG ; C.S. DIN ; S. MILLER ; S.A. NATHOO ; A. GAFFAR**. Intra-oral remineralization of enamel with a MFP/DCPD and MFP/silica dentifrice using surface microhardness. *J Clin Dent*, 1995, vol. 6 (2), 148-153
- **R.J. SULLIVAN ; A. CHARIG ; J. BLAKE-HASKINS ; Y.P. ZHANG ; S.M. MILLER ; M. STRANNICK ; A. GAFFAR ; H.C. MARGOLIS**. In-vivo detection of calcium from dicalcium phosphate dihydrate dentifrices in demineralized human enamel and plaque. *Adv Dent Res*, 1997, vol. 11 (4), 380-387
- **A.K. BURWELL ; L.J. LITKOWSKI ; D.C. GREENSPAN**. *Calcium sodium phosphosilicate (NovaMin®): remineralization potential \\ Adv Dent Res*, 2009, vol. 21 (1), 35-39
- **D.M. DA CAMARA ; T.M. FRANCATI ; J.A.S. SOUZA ; M. DANELON ; A.C.B. DELBEM**. Fluoride tooth paste supplemented with sodium hexametaphosphate reduces enamel demineralization in-vitro. *Clin Oral Investig*, 2016, vol. 20 (8), 1981-1985
- **E.M. TAKESHITA ; M. DANELON ; L.P. CASTRO ; R.F. CUNHA ; A.C.B. DELBEM**. Remineralizing potential of a low fluoride tooth paste with sodium trimetaphosphate: an in-situ study. *Caries Res*, 2016, vol. 50 (6), 571-578

- **A.C.S.F. ZAZE** ; **A.P. DIAS** ; **K.T. SASSAKI** ; **A.C.B. DELBEM**. The effects of low-fluoride tooth paste supplemented with calcium glycerophosphate on enamel demineralization. *Clin Oral Investig*, 2014, vol. 18 (6), 1619-1624
- **I.R. FREIRE** ; **J.P. PESSAN** ; **J.G. AMARAL** ; **C.C. MARTINHON** ; **R.F. CUNHA** ; **A.C.B. DELBEM**. Anticaries effect of low-fluoride dentifrices with phosphates in children: a randomized controlled trial. *J Dent*, 2016, vol. 50, 37-42
- **E.M. TAKESHITA** ; **R.A. EXTERKATE** ; **A.C.B. DELBEM**. J.M. Ten Cate. Evaluation of different fluoride concentrations supplemented with trimetaphosphate on enamel de- and remineralization in-vitro. *Caries Res*, 2011, vol. 45 (5), 494-497
- **HU, D.** ; **REN, Q** ; **LI, Z.** ; **ZHANG, L.** Chitosan-Based Biomimetically Mineralized Composite Materials in Human Hard Tissue Repair. *Molecules*, 2020, vol. 25, 4785
- **CICCIÙ, M.** ; **FIORILLO, L.** ; **CERVINO, G.** Chitosan Use in Dentistry: A Systematic Review of Recent Clinical Studies. *Mar. Drugs*, 2019, vol. 17, 417
- **AKSHAT GUPTA** ; **VIDYA RATTAN** ; **SACHIN RAI**. Efficacy of Chitosan in promoting wound healing in extraction socket: A prospective study. *Journal of Oral Biology and Craniofacial Research*, 2019, vol. 9 (1), 91-95
- **HARUN ACHMAD** ; **YUNITA FEBY RAMADHANY**. Effectiveness of Chitosan Tooth Paste from White Shrimp (Litopenaeusvannamei) to Reduce Number of Streptococcus Mutans in the Case of Early Childhood Caries. *Journal of International Dental and Medical Research.*, 2017, vol. 10 (2), 358-363
- **KMIEC M** ; **PIGHINELLI L** ; **TEDESCO MF et al.** Chitosan-properties and applications in dentistry. *Adv Tissue Eng Regen Med Open Access.*, 2017, vol. 2 (4), 205-211
- **THATIANA M** ; **STAMFORD ARNAUD** ; **BENÍCIO DE BARROS NETO** ; **FLAMARION B. DINIZ.** Chitosan effect on dental enamel de-remineralization: An in vitro evaluation. *Journal of Dentistry*, 2010, vol. 38 (11), 848-852
- **FAKHRI, E.** ; **ESLAMI, H.** ; **MAROUFI, P.** ; **PAKDEL, F.** ; **TAGHIZADEH, S.** ; **GANBAROV, K.** ; **KAFIL, H. S.** Chitosan biomaterials application in dentistry. *International Journal of Biological Macromolecules*, 2020
- **SHUN-YI YAO** ; **SI-PENG CHEN** ; **RUO-XUN WANG** ; **KAI ZHANG** ; **XIAO-XUAN LIN** ; **SUI MAI**. Antibacterial activity and bonding performance of carboxymethyl chitosan-containing dental adhesive system. *International Journal of Adhesion and Adhesives*, 2022, vol. 119
- **CAI, M.** ; **RATNAYAKE, J.** ; **CATHRO, P.** ; **GOULD, M.** ; **ALI, A.** Investigation of a Novel Injectable Chitosan Oligosaccharide-Bovine Hydroxyapatite Hybrid Dental Biocomposite for the Purposes of Conservative Pulp Therapy. *Nanomaterials*, 2022, vol. 12, 3925
- **ZAHRA SHARIATINIA**. Carboxymethyl chitosan: Properties and biomedical applications. *International Journal of Biological Macromolecules*, 2018, vol. 120, 1406-1419
- **KUMAR, M. N. V. R.** ; **MUZZARELLI, R. A. A.** ; **MUZZARELLI, C.** ; **SASHIWA, H.** ; **DOMB, A. J.** Chitosan Chemistry and Pharmaceutical Perspectives. *Chemical Reviews*, 2004, vol. 104 (12), 6017-6084
- **DALIA Y. ZAKI** ; **ENGIE M. SAFWAT** ; **SHAYMAA M. NAGI** ; **HAIDY N. SALEM** ; **TAMER M. HAMDY** ; **LAMIAA M. MOHARAM** ; **MOHAMMAD L. HASSAN** ; **E.M.A. HAMZAWY**. A novel dental remineralizing blend of hydroxyethyl-cellulose and cellulose nanofibers oral film loaded with nepheline apatite glass: Preparation, characterization and in vitro evaluation of re-mineralizing effect. *Carbohydrate Polymer Technologies and Applications*, 2021, vol. 2
- **FATULLAYEVA, S.** ; **TAGIYEV, D.** ; **ZEYNALOV, N. et al.** Recent advances of chitosan-based polymers in biomedical applications and environmental protection. *J Polym Res*, 2022, vol. 29, 259
- **E. PEETERS** ; **H.J. NELIS** ; **T. COENYE**. Comparison of multiple methods for quantification of microbial biofilms grown in microtiter plates. *Journal of Microbiological MethodS.*, 2008, vol. 72 (2), 157-165
- **J. SAMBROOK** ; **E. F. FRITSCH** ; **T. MANIATIS**. Molecular cloning: a laboratory manual [Text. Cold Spring Harbor Laboratory PresS, 1989
- **I.F.F. BENZIE** ; **J.J. STRAIN**. The ferric reducing ability of plasma: FRAP as a measure of ''antioxidant power'' the FRAP assay. *Anal. Biochem.*, 1996, vol. 239, 70-76
- **IORGULESCU G**. Saliva between normal and pathological. Important factors in determining systemic and oral health. *J Med Life.*, 2009, vol. 2 (3), 303-307